(19) Europäisches Patentamt
European Patent Office
Office européen des brevets

(11) Veröffentlichungsnummer: **0 200 872**
**B1**

(12) **EUROPÄISCHE PATENTSCHRIFT**

(45) Veröffentlichungstag der Patentschrift: **18.04.90**

(51) Int. Cl.$^5$: **C 07 D 231/16,** A 01 N 43/56

(21) Anmeldenummer: **86102868.6**

(22) Anmeldetag: **05.03.86**

(54) **Herbizide und insektizide Mittel auf Basis von Pyrazol-Derivaten.**

(30) Priorität: **16.03.85 DE 3509567**

(43) Veröffentlichungstag der Anmeldung:
**12.11.86 Patentblatt 86/46**

(45) Bekanntmachung des Hinweises auf die Patenterteilung:
**18.04.90 Patentblatt 90/16**

(84) Benannte Vertragsstaaten:
**AT BE CH DE FR GB IT LI NL SE**

(56) Entgegenhaltungen:
**EP-A-0 139 182**
**US-A-3 869 274**

(73) Patentinhaber: **BAYER AG**
**D-5090 Leverkusen 1 Bayerwerk (DE)**

(72) Erfinder: **Gehring, Reinhold, Dr.**
**Dasnöckel 49**
**D-5600 Wuppertal 11 (DE)**
Erfinder: **Jensen-Korte, Uta, Dr.**
**Geibelstrasse 9**
**D-4000 Düsseldorf (DE)**
Erfinder: **Schallner, Otto, Dr.**
**Noldeweg 22**
**D-4019 Monheim (DE)**
Erfinder: **Stetter, Jörg, Dr.**
**Gellertweg 4**
**D-5600 Wuppertal 1 (DE)**
Erfinder: **Santel, Hans-Joachim, Dr.**
**Grünstrasse 9a**
**D-5090 Leverkusen (DE)**
Erfinder: **Schmidt, Robert R., Dr.**
**Im Waldwinkel 110**
**D-5060 Bergisch-Gladbach 2 (DE)**
Erfinder: **Behrenz, Wolfgang, Dr.**
**Untergründemich 14**
**D-5063 Overath Steinenbrück (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 200 872 B1

**Beschreibung**

Die Erfindung betrifft die Verwendung von teilweise bekannten 4-Nitro-1-phenyl-pyrazolen als Herbizide und Insektizide.

Es ist bereits bekannt, daß bestimmte in 4-Stellung durch eine Cyano-Gruppe substituierte 5-Amino-1-phenyl-pyrazole, wie beispielsweise das 4-Cyano-5-propionamido-1-(2,4,6-trichlorphenyl)-pyrazol herbizide Eigenschaften besitzen (vgl. z.B. DE—OS 3 226 513).

Deren herbizide Wirksamkeit gegenüber einigen Problemunkräutern ebenso wie ihre Verträglichkeit gegenüber wichtigen Nutzpflanzen ist jedoch nicht immer in allen Anwendungsbereichen völlig zufriedenstellend.

Außerdem ist bekannt, daß Pyrazole, wie beispielsweise 5-Dimethylaminocarbonyloxy-1-isopropyl-3-methylsulfinylmethylpyrazol oder 1-Cyclohexyl-5-dimethylaminocarbonyloxy-3-methylthiomethyl-pyrazol insektizide Eigenschaften besitzen (vgl. DE—OS 2 819 932 und DE—OS 2 839 270).

Deren Wirkung ist jedoch insbesondere bei niedrigen Aufwandmengen oder -konzentrationen nicht immer gegenüber allen Schadinsekten völlig zufriedenstellend.

Weiterhin sind einige 4-Nitro-1-phenylpyrazole, wie beispielsweise das 4-Nitro-1-(2,4,6-trinitrophenyl)-pyrazol, das 4-Nitro-1-(2,4-dichlorphenyl)-pyrazol, das 4-Nitro-1-pentafluorphenylpyrazol, das 3-Methyl-4-nitro-1-(2,4-dinitrophenyl)-pyrazol oder das 3,5-Dimethyl-4-nitro-1-(2,4-dinitrophenyl)-pyrazol bekannt [vgl. z.B. Bull, Soc. Chim, France *1966,* 3727—3743; J. Chem. Soc. *B, 1968,* 211—214; J. Heterocycl. Chem, *7,* 345—349 (1970); Farmaco Ed. Sci. *21,* 883—891 (1966) bzw. C.A. *66;* 115 640 p].

Über eine herbizide oder insektizide Wirksamkeit dieser vorbekannten Verbindungen ist jedoch nichts bekannt.

Es wurde nun gefunden, daß die teilsweise bekannten 4-Nitro-1-phenyl-pyrazole der allgemeinen Formel (I),

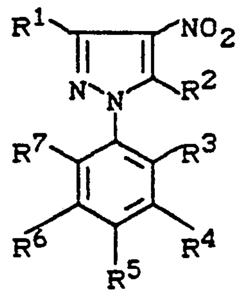

(I)

in welcher

$R^1$ und $R^2$ jeweils unabhängig voneinander für Wasserstoff oder für jeweils geradkettiges oder verzweigtes Alkyl, Hydroxyalkyl, Alkoxyalkyl, Alkylthioalkyl oder Halogenalkyl mit jeweils bis zu 4 Kohlenstoffatomen in den einzelnen Alkylteilen und gegebenenfalls bis zu 9 gleichen oder verschiedenen Halogenatomen oder für Cycloalkyl mit 3 bis 7 Kohlenstoffatomen stehen und

$R^3$, $R^4$, $R^5$, $R^6$ und $R^7$ jeweils unabhängig voneinander für Wasserstoff, Fluor, Chlor, Brom, Jod, Cyano, Nitro, jeweils geradkettiges oder verzweigtes Alkyl, Alkoxy, Alkylthio, Alkylsulfinyl, Alkylsulfonyl oder Alkoxycarbonyl mit jeweils bis zu 4 Kohlenstoffatomen in den einzelnen Alkylteilen oder für einen Rest $—(X)_n—R^8$ stehen, wobei

X für Sauerstoff, Schwefel, Sulfinyl oder Sulfonyl steht,

n für eine Zahl 0 oder 1 steht und

$R^8$ für geradkettiges oder verzweigtes Halogenalkyl mit biz zu 4 Kohlenstoffatomen und bis zu 9 gleichen oder verschiedenen Halogenatomen steht, herbizide Eigenschaften, insbesondere auch selektiv herbizide Eigenschaften und darüber hinaus auch insektizide Eigenschaften besitzen.

Überraschenderweise zeigen die erfindungsgemäß verwendbaren 4-Nitro-1-phenyl-pyrazole der allgemeinen Formel (I) neben einer erheblich verbesserten herbiziden Wirksamkeit gegenüber Schadpflanzen auch eine deutlich verbesserte Verträglichkeit gegenüber wichtigen Kulturpflanzen als die aus dem Stand der Technik bekannten 1-Phenylpyrazole, wie beispielsweise das 4-Cyano-5-propionamido-1-(2,4,6-trichlorphenyl)-pyrazol, welche chemisch und wirkungsmäßig naheliegende Verbindungen sind, Darüber hinaus zeigen die erfindungsgemäß verwendbaren 4-Nitro-1-phenyl-pyrazole der allgemeinen Formel (I) völlig unerwartet auch eine insektizide Wirksamkeit. Bevorzugt verwendet man Verbindungen der allgemeinen Formel (I), bei welchen

$R^1$ und $R^2$ unabhängig voneinander für Wasserstoff, Methyl, Ethyl, n- oder i-Propyl, n-, i-, s-, oder t-Butyl, Cyclohexyl, Hydroxymethyl, Methoxymethyl, Methylthiomethyl, Trifluormethyl oder Trichlormethyl stehen und

$R^3$, $R^4$, $R^5$, $R^6$ und $R^7$ jeweils unabhängig voneinander für Wasserstoff, Fluor, Chlor, Brom, Jod, Cyano, Nitro, Methyl, Ethyl, n- und i-Propyl, Methoxy, Ethoxy, Methylthio, Methylsulfinyl, Methylsulfonyl, Methoxycarbonyl, Ethoxycarbonyl oder für einen Rest $—(X)_n—R^8$ stehen, wobei

X für Sauerstoff, Schwefel, Sulfinyl oder Sulfonyl steht,

n für 0 oder 1 steht und

$R^8$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl steht.

Im einzelnen seien außer den bei den Herstellungsbeispielen genannten Verbindungen, die folgenden Verbindungen der allgemeinen Formel (I) genannt:

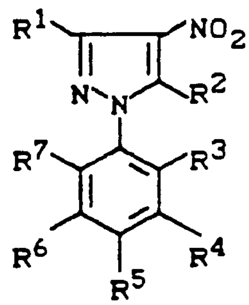

(I)

Tabelle 1:

| $R^1$ | $R^2$ | $R^3$ | $R^4$ | $R^5$ | $R^6$ | $R^7$ |
|---|---|---|---|---|---|---|
| H | H | H | H | $CF_3$ | H | H |
| H | H | Cl | H | $CF_3$ | H | H |
| H | H | Cl | H | $CF_3$ | H | Cl |
| H | H | Cl | Cl | $CF_3$ | H | Cl |
| H | H | F | H | $CF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | F | H | $CF_3$ | H | F |
| H | H | F | F | $CF_3$ | F | F |
| H | H | Cl | Cl | $CF_3$ | H | H |
| H | H | Cl | F | $CF_3$ | F | Cl |
| H | H | Cl | F | $CF_3$ | F | F |
| H | H | F | F | CN | F | F |
| H | H | Cl | H | Br | H | Cl |
| H | H | Cl | F | CN | F | F |
| H | H | Cl | F | CN | F | Cl |
| $CH_3$ | H | Cl | F | $CF_3$ | F | Cl |
| $CH_3$ | H | Cl | F | $CF_3$ | F | F |
| $CH_3$ | H | F | F | CN | F | F |
| $CH_3$ | H | Cl | F | CN | F | F |
| $CH_3$ | H | Cl | F | CN | F | Cl |
| $CH_3$ | H | Cl | H | Br | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Cl | F | $CF_3$ | F | Cl |
| H | $CH_3$ | Cl | F | $CF_3$ | F | F |
| H | $CH_3$ | F | F | CN | F | F |
| H | $CH_3$ | Cl | F | CN | F | F |
| H | $CH_3$ | Cl | F | CN | F | Cl |
| H | $CH_3$ | Cl | H | Br | H | Cl |
| $CH_3$ | $CH_3$ | Cl | F | $CF_3$ | F | Cl |
| $CH_3$ | $CH_3$ | Cl | F | $CF_3$ | F | F |
| $CH_3$ | $CH_3$ | F | F | CN | F | F |
| $CH_3$ | $CH_3$ | Cl | F | CN | F | F |
| $CH_3$ | $CH_3$ | Cl | F | CN | F | Cl |
| $CH_3$ | $CH_3$ | Cl | H | Br | H | Cl |
| H | H | Cl | H | $CF_3$ | H | F |
| H | H | Br | H | $CF_3$ | H | H |
| H | H | Br | H | $CF_3$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | H | H | H | $CF_3$ | H | H |
| $CH_3$ | H | Cl | H | $CF_3$ | H | H |
| $CH_3$ | H | Cl | H | $CF_3$ | H | Cl |
| $CH_3$ | H | Cl | Cl | $CF_3$ | H | Cl |
| $CH_3$ | H | F | H | $CF_3$ | H | H |
| $CH_3$ | H | F | H | $CF_3$ | H | F |
| $CH_3$ | H | F | F | $CF_3$ | F | F |
| $CH_3$ | H | Cl | Cl | $CF_3$ | H | H |
| $CH_3$ | H | Cl | H | $CF_3$ | H | F |
| $CH_3$ | H | Br | Br | $CF_3$ | H | H |
| $CH_3$ | H | Br | Br | $CF_3$ | H | Br |
| $CH_3$ | $CH_3$ | H | H | $CF_3$ | H | H |
| $CH_3$ | $CH_3$ | Cl | H | $CF_3$ | H | H |
| $CH_3$ | $CH_3$ | Cl | H | $CF_3$ | H | Cl |
| $CH_3$ | $CH_3$ | Cl | Cl | $CF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | F | H | $CF_3$ | H | H |
| $CH_3$ | $CH_3$ | F | H | $CF_3$ | H | F |
| $CH_3$ | $CH_3$ | F | F | $CF_3$ | F | F |
| $CH_3$ | $CH_3$ | Cl | Cl | $CF_3$ | H | H |
| $CH_3$ | $CH_3$ | Cl | H | $CF_3$ | H | F |
| $CH_3$ | $CH_3$ | Br | H | $CF_3$ | H | Br |
| H | $CH_3$ | H | H | $CF_3$ | H | H |
| H | $CH_3$ | Cl | H | $CF_3$ | H | H |
| H | $CH_3$ | Cl | H | $CF_3$ | H | Cl |
| H | $CH_3$ | Cl | Cl | $CF_3$ | H | Cl |
| H | $CH_3$ | F | H | $CF_3$ | H | H |
| H | $CH_3$ | F | H | $CF_3$ | H | F |
| H | $CH_3$ | F | F | $CF_3$ | F | F |
| H | $CH_3$ | Cl | Cl | $CF_3$ | H | H |
| H | H | Cl | H | $CF_3$ | H | F |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Br | H | $CF_3$ | H | H |
| H | $CH_3$ | Br | H | $CF_3$ | H | Br |
| H | H | F | H | $OCF_3$ | H | H |
| H | H | F | H | $OCF_3$ | H | F |
| H | H | F | F | $OCF_3$ | F | F |
| H | H | Cl | H | $OCF_3$ | H | H |
| H | H | Cl | H | $OCF_3$ | H | Cl |
| H | H | Cl | Cl | $OCF_3$ | H | H |
| H | H | Cl | Cl | $OCF_3$ | H | Cl |
| H | H | Br | H | $OCF_3$ | H | H |
| H | H | Br | H | $OCF_3$ | H | Br |
| H | $CH_3$ | F | H | $OCF_3$ | H | H |
| H | $CH_3$ | F | H | $OCF_3$ | H | F |
| H | $CH_3$ | F | F | $OCF_3$ | F | F |
| H | $CH_3$ | Cl | H | $OCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Cl | H | $OCF_3$ | H | Cl |
| H | $CH_3$ | Cl | Cl | $OCF_3$ | H | H |
| H | $CH_3$ | Cl | Cl | $OCF_3$ | H | Cl |
| H | $CH_3$ | Br | H | $OCF_3$ | H | H |
| H | $CH_3$ | Br | H | $OCF_3$ | H | Br |
| $CH_3$ | H | F | H | $OCF_3$ | H | H |
| $CH_3$ | H | F | H | $OCF_3$ | H | F |
| $CH_3$ | H | F | F | $OCF_3$ | F | F |
| $CH_3$ | H | Cl | H | $OCF_3$ | H | H |
| $CH_3$ | H | Cl | H | $OCF_3$ | H | Cl |
| $CH_3$ | H | Cl | Cl | $OCF_3$ | H | H |
| $CH_3$ | H | Cl | Cl | $OCF_3$ | H | Cl |
| $CH_3$ | H | Br | H | $OCF_3$ | H | H |
| $CH_3$ | H | Br | H | $OCF_3$ | H | Br |
| $CH_3$ | $CH_3$ | F | H | $OCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | F | H | $OCF_3$ | H | F |
| $CH_3$ | $CH_3$ | F | F | $OCF_3$ | F | F |
| $CH_3$ | $CH_3$ | Cl | H | $OCF_3$ | H | H |
| $CH_3$ | $CH_3$ | Cl | H | $OCF_3$ | H | Cl |
| $CH_3$ | $CH_3$ | Cl | Cl | $OCF_3$ | H | H |
| $CH_3$ | $CH_3$ | Cl | Cl | $OCF_3$ | H | Cl |
| $CH_3$ | $CH_3$ | Br | H | $OCF_3$ | H | H |
| $CH_3$ | $CH_3$ | Br | H | $OCF_3$ | H | Br |
| H | $CH_3$ | Cl | H | $-S-CH_2CF_3$ | H | H |
| H | $CH_3$ | Cl | H | $-S-CH_2CF_3$ | H | Cl |
| H | CH3 | Br | H | $-S-CH_2CF_3$ | H | H |
| H | $CH_3$ | Br | H | $-S-CH_2CF_3$ | H | Br |
| H | $CH_3$ | Cl | Cl | $-S-CH_2CF_3$ | H | H |
| $CH_3$ | H | Cl | H | $-S-CH_2CF_3$ | H | H |
| $CH_3$ | CH3 | Cl | H | $-S-CH_2CF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | Br | H | $-S-CH_2CF_3$ | H | H |
| $CH_3$ | $CH_3$ | Br | H | $-S-CH_2CF_3$ | H | Br |
| $CH_3$ | $CH_3$ | Cl | Cl | $-S-CH_2CF_3$ | H | H |
| H | $CH_3$ | Cl | H | $-S-CH_2CF_3$ | H | H |
| H | $CH_3$ | Cl | H | $-S-CH_2CF_3$ | H | Cl |
| H | $CH_3$ | Br | H | $-S-CH_2CF_3$ | H | H |
| H | $CH_3$ | Br | H | $-S-CH_2CF_3$ | H | Br |
| H | $CH_3$ | Cl | Cl | $-S-CH_2CF_3$ | H | H |
| $CH_3$ | $CH_3$ | Cl | H | $-S-CH_2CF_3$ | H | H |
| $CH_3$ | $CH_3$ | Cl | H | $-S-CH_2CF_3$ | H | Cl |
| $CH_3$ | $CH_3$ | Br | H | $-S-CH_2CF_3$ | H | H |
| $CH_3$ | $CH_3$ | Br | H | $-S-CH_2CF_3$ | H | Br |
| $CH_3$ | CH3 | Cl | Cl | $-S-CH_2CF_3$ | H | H |
| H | $CH_3$ | Cl | H | $-O-CH_2CF_3$ | H | H |
| H | $CH_3$ | Cl | H | $-O-CH_2CF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Br | H | $-O-CH_2CF_3$ | H | H |
| H | $CH_3$ | Br | H | $-O-CH_2CF_3$ | H | Br |
| H | H | Cl | H | $-O-CH_2CF_3$ | H | H |
| H | H | Cl | H | $-O-CH_2CF_3$ | H | Cl |
| H | H | Br | H | $-O-CH_2CF_3$ | H | H |
| H | H | Br | H | $-O-CH_2CF_3$ | H | Br |
| $CH_3$ | H | Cl | H | $-O-CH_2CF_3$ | H | H |
| $CH_3$ | H | Cl | H | $-O-CH_2CF_3$ | H | Cl |
| $CH_3$ | H | Br | H | $-O-CH_2CF_3$ | H | H |
| $CH_3$ | H | Br | H | $-O-CH_2CF_3$ | H | Br |
| $CH_3$ | $CH_3$ | Cl | H | $-O-CH_2CF_3$ | H | H |
| $CH_3$ | $CH_3$ | Cl | H | $-O-CH_2CF_3$ | H | Cl |
| $CH_3$ | $CH_3$ | Br | H | $-O-CH_2CF_3$ | H | H |
| $CH_3$ | $CH_3$ | Br | H | $-O-CH_2CF_3$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | $SCF_3$ | H | Cl | H | H |
| H | $CH_3$ | $SCF_3$ | H | Cl | H | H |
| H | H | $SCF_3$ | H | Cl | H | Cl |
| H | $CH_3$ | $SCF_3$ | H | Cl | H | Cl |
| H | H | F | H | $SCF_3$ | H | H |
| H | H | F | H | $SCF_3$ | H | F |
| H | H | F | F | $SCF_3$ | F | F |
| H | H | Cl | H | $SCF_3$ | H | H |
| H | H | Cl | H | $SCF_3$ | H | Cl |
| H | H | Cl | Cl | $SCF_3$ | H | H |
| H | H | Cl | Cl | $SCF_3$ | H | Cl |
| H | H | Br | H | $SCF_3$ | H | H |
| H | H | Br | H | $SCF_3$ | H | Br |
| H | $CH_3$ | F | H | $SCF_3$ | H | H |
| H | $CH_3$ | F | H | $SCF_3$ | H | F |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | F | F | $SCF_3$ | F | F |
| H | $CH_3$ | Cl | H | $SCF_3$ | H | H |
| H | $CH_3$ | Cl | H | $SCF_3$ | H | Cl |
| H | $CH_3$ | Cl | Cl | $SCF_3$ | H | H |
| H | $CH_3$ | Cl | Cl | $SCF_3$ | H | Cl |
| H | $CH_3$ | Br | H | $SCF_3$ | H | H |
| H | $CH_3$ | Br | H | $SCF_3$ | H | Br |
| $CH_3$ | H | F | H | $SCF_3$ | H | H |
| $CH_3$ | H | F | H | $SCF_3$ | H | F |
| $CH_3$ | H | F | H | $SCF_3$ | F | F |
| $CH_3$ | H | Cl | H | $SCF_3$ | H | H |
| $CH_3$ | H | Cl | H | $SCF_3$ | H | Cl |
| $CH_3$ | H | Cl | H | $SCF_3$ | H | F |
| $CH_3$ | H | Br | H | $SCF_3$ | H | H |
| $CH_3$ | $CH_3$ | F | H | $SCF_3$ | H | H |
| $CH_3$ | $CH_3$ | F | H | $SCF_3$ | H | F |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | F | F | $SCF_3$ | F | F |
| $CH_3$ | $CH_3$ | Cl | H | $SCF_3$ | H | H |
| $CH_3$ | $CH_3$ | Cl | H | $SCF_3$ | H | Cl |
| $CH_3$ | $CH_3$ | Cl | Cl | $SCF_3$ | H | H |
| $CH_3$ | $CH_3$ | Cl | Cl | $SCF_3$ | H | Cl |
| $CH_3$ | CH3 | Br | H | $SCF_3$ | H | H |
| $CH_3$ | $CH_3$ | Br | H | $SCF_3$ | H | Br |
| H | H | $CF_3$ | H | $-SO_2CH_3$ | H | H |
| H | H | $CF_3$ | H | $-SCF_3$ | H | H |
| H | H | $OCF_3$ | H | $CF_3$ | H | H |
| H | H | $OCF_3$ | H | $-OCF_3$ | H | H |
| H | $CH_3$ | $CF_3$ | H | $-SO_2CH_3$ | H | H |
| H | $CH_3$ | $CF_3$ | H | $-SO_2CH_3$ | H | H |
| H | $CH_3$ | $CF_3$ | H | $-SCF_3$ | H | H |
| H | $CH_3$ | $OCF_3$ | H | $-OCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | $OCF_3$ | H | $-CF_3$ | H | H |
| $CH_3$ | H | $CF_3$ | H | $-SO_2CH_3$ | H | H |
| $CH_3$ | H | $CF_3$ | H | $-SO_2CH_3$ | H | H |
| $CH_3$ | H | $CF_3$ | H | $-SCF_3$ | H | H |
| $CH_3$ | H | $OCF_3$ | H | $-OCF_3$ | H | H |
| $CH_3$ | H | $OCF_3$ | H | $-CF_3$ | H | H |
| $CH_3$ | $CH_3$ | $CF_3$ | H | $-SO_2CH_3$ | H | H |
| $CH_3$ | $CH_3$ | $CF_3$ | H | $-SO_2CH_3$ | H | H |
| $CH_3$ | $CH_3$ | $CF_3$ | H | $-SCF_3$ | H | H |
| $CH_3$ | $CH_3$ | $OCF_3$ | H | $-OCF_3$ | H | H |
| $CH_3$ | $CH_3$ | $OCF_3$ | H | $-CF_3$ | H | H |
| H | H | Cl | H | $-SCHF_2$ | H | H |
| H | H | Cl | H | $-SCHF_2$ | H | Cl |
| H | H | Br | H | $-SCHF_2$ | H | H |
| H | H | Br | H | $-SCHF_2$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Cl | H | $-SCHF_2$ | H | H |
| H | $CH_3$ | Cl | H | $-SCHF_2$ | H | Cl |
| H | $CH_3$ | Br | H | $-SCHF_2$ | H | H |
| H | $CH_3$ | Br | H | $-SCHF_2$ | H | Br |
| H | H | Cl | H | $-SCHF_2$ | H | H |
| H | H | Cl | H | $-SCHF_2$ | H | Cl |
| H | H | Br | H | $-SCHF_2$ | H | H |
| H | H | Br | H | $-SCHF_2$ | H | Br |
| $CH_3$ | H | Cl | H | $-SCHF_2$ | H | H |
| $CH_3$ | H | Cl | H | $-SCHF_2$ | H | Cl |
| $CH_3$ | H | Br | H | $-SCHF_2$ | H | H |
| $CH_3$ | H | Br | H | $-SCHF_2$ | H | Br |
| H | H | Cl | H | $-SCF_2CHF_2$ | H | H |
| H | H | Cl | H | $-SCF_2CHF_2$ | H | Cl |
| H | H | Br | H | $-SCF_2CHF_2$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | Br | H | $-SCF_2CHF_2$ | H | Br |
| H | $CH_3$ | Cl | H | $-SCF_2CHF_3$ | H | H |
| H | $CH_3$ | Cl | H | $-SCF_2CHF_2$ | H | Cl |
| H | $CH_3$ | Br | H | $-SCF_2CHF_2$ | H | H |
| H | $CH_3$ | Br | H | $-SCF_2CHF_2$ | H | Br |
| $CH_3$ | H | Cl | H | $-SCF_2CHF_2$ | H | H |
| $CH_3$ | H | Cl | H | $-SCF_2CHF_2$ | H | Cl |
| $CH_3$ | H | Br | H | $-SCF_2CHF_2$ | H | H |
| $CH_3$ | H | Br | H | $-SCF_2CHF_2$ | H | Br |
| $CH_3$ | $CH_3$ | Cl | H | $-SCF_2CHF_2$ | H | H |
| $CH_3$ | $CH_3$ | Cl | H | $-SCF_2CHF_2$ | H | Cl |
| $CH_3$ | $CH_3$ | Br | H | $-SCF_2CHF_2$ | H | H |
| $CH_3$ | $CH_3$ | Br | H | $-SCF_2CHF_2$ | H | Br |
| H | H | Cl | H | $-SCF_2CHFCl$ | H | H |
| H | H | Cl | H | $-SCF_2CHFCl$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | Br | H | $-SCF_2CHFCl$ | H | H |
| H | H | Br | H | $-SCF_2CHFCl$ | H | Br |
| H | $CH_3$ | Cl | H | $-SCF_2CHFCl$ | H | H |
| H | $CH_3$ | Cl | H | $-SCF_2CHFCl$ | H | Cl |
| H | $CH_3$ | Br | H | $-SCF_2CHFCl$ | H | H |
| H | $CH_3$ | Br | H | $-SCF_2CHFCl$ | H | Br |
| $CH_3$ | H | Cl | H | $-SCF_2CHFCl$ | H | H |
| $CH_3$ | H | Cl | H | $-SCF_2CHFCl$ | H | Cl |
| $CH_3$ | H | Cl | H | $-SCF_2CHFCl$ | H | Cl |
| $CH_3$ | H | Br | H | $-SCF_2CHFCl$ | H | H |
| $CH_3$ | H | Br | H | $-SCF_2CHFCl$ | H | Br |
| $CH_3$ | $CH_3$ | Cl | H | $-SCF_2CHFCl$ | H | H |
| $CH_3$ | $CH_3$ | Cl | H | $-SCF_2CHFCl$ | H | Cl |
| $CH_3$ | $CH_3$ | Br | H | $-SCF_2CHFCl$ | H | H |
| $CH_3$ | $CH_3$ | Br | H | $-SCF_2CHFCl$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $C_2H_5$ | H | Cl | H | $-SCF_3$ | H | H |
| $C_2H_5$ | H | Cl | H | $-OCF_3$ | H | Cl |
| $C_2H_5$ | H | Br | H | $-OCF_3$ | H | H |
| $C_2H_5$ | H | Br | H | $-OCF_3$ | H | Br |
| $C_2H_5$ | $CH_3$ | Cl | H | $-OCF_3$ | H | H |
| $C_2H_5$ | $CH_3$ | Cl | H | $-OCF_3$ | H | Cl |
| $C_2H_5$ | H | Cl | H | $-SCF_3$ | H | H |
| $C_2H_5$ | H | Cl | H | $-SCF_3$ | H | Cl |
| $C_2H_5$ | H | Br | H | $-SCF_3$ | H | H |
| H | H | Cl | H | $-SOCF_3$ | H | H |
| H | H | Cl | H | $-SOCF_3$ | H | Cl |
| H | H | Br | H | $-SOCF_3$ | H | Br |
| H | H | Br | H | $-SOCF_3$ | H | H |
| H | H | $CF_3$ | H | $-SOCF_3$ | H | H |
| H | H | $CF_3$ | H | $-SOCF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $CH_3$ | Cl | H | $-SOCF_3$ | H | H |
| H | $CH_3$ | Cl | H | $-SOCF_3$ | H | Cl |
| H | $CH_3$ | Br | H | $-SOCF_3$ | H | Br |
| H | $CH_3$ | Br | H | $-SOCF_3$ | H | H |
| H | $CH_3$ | $CF_3$ | H | $-SOCF_3$ | H | H |
| H | $CH_3$ | $CF_3$ | H | $-SOCF_3$ | H | Cl |
| $CH_3$ | H | Cl | H | $-SOCF_3$ | H | H |
| $CH_3$ | H | Cl | H | $-SOCF_3$ | H | Cl |
| $CH_3$ | H | Br | H | $-SOCF_3$ | H | Br |
| $CH_3$ | H | Br | H | $-SOCF_3$ | H | H |
| $CH_3$ | H | $CF_3$ | H | $-SOCF_3$ | H | H |
| $CH_3$ | H | $CF_3$ | H | $-SOCF_3$ | H | Cl |
| $CH_3$ | $CH_3$ | Cl | H | $-SOCF_3$ | H | H |
| $CH_3$ | $CH_3$ | Cl | H | $-SOCF_3$ | H | Cl |
| $CH_3$ | $CH_3$ | Br | H | $-SOCF_3$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | Br | H | $-SOCF_3$ | H | H |
| $CH_3$ | $CH_3$ | $CF_3$ | H | $-SOCF_3$ | H | H |
| $CH_3$ | $CH_3$ | $CF_3$ | H | $-SOCF_3$ | H | Cl |
| H | H | Cl | H | $-OCF_2CHFCl$ | H | H |
| H | H | Cl | H | $-OCF_2CHFCl$ | H | Cl |
| H | H | Br | H | $-OCF_2CHFCl$ | H | H |
| H | H | Cl | H | $-OCF_2CHFCl$ | H | Br |
| H | $CH_3$ | Cl | H | $-OCF_2CHFCl$ | H | H |
| H | H | Cl | H | $-OCF_2CHFCl$ | H | Cl |
| H | H | Br | H | $-OCF_2CHFCl$ | H | H |
| H | H | Br | H | $-OCF_2CHFCl$ | H | Br |
| $CH_3$ | H | Cl | H | $-OCF_2CHFCl$ | H | H |
| $CH_3$ | H | Cl | H | $-OCF_2CHFCl$ | H | Cl |
| $CH_3$ | H | Br | H | $-OCF_2CHFCl$ | H | H |
| $CH_3$ | H | Br | H | $-OCF_2CHFCl$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | Cl | H | $-OCF_2CHFCl$ | H | H |
| $CH_3$ | $CH_3$ | Cl | H | $-OCF_2CHFCl$ | H | Cl |
| $CH_3$ | $CH_3$ | Br | H | $-OCF_2CHFCl$ | H | H |
| $CH_3$ | $CH_3$ | Br | H | $-OCF_2CHFCl$ | H | Br |
| H | H | Cl | H | $-OCF_2CHCl_2$ | H | H |
| H | H | Cl | H | $-OCF_2CHCl_2$ | H | Cl |
| H | H | Br | H | $-OCF_2CHCl_2$ | H | H |
| H | H | Br | H | $-OCF_2CHCl_2$ | H | Br |
| H | $CH_3$ | Cl | H | $-OCF_2CHCl_2$ | H | H |
| H | $CH_3$ | Cl | H | $-OCF_2CHCl_2$ | H | Cl |
| H | $CH_3$ | Br | H | $-OCF_2CHCl_2$ | H | H |
| H | $CH_3$ | Br | H | $-OCF_2CHCl_2$ | H | Br |
| $CH_3$ | H | Cl | H | $-OCF_2CHCl_2$ | H | H |
| $CH_3$ | H | Cl | H | $-OCF_2CHCl_2$ | H | Cl |
| $CH_3$ | H | Br | H | $-OCF_2CHCl_2$ | H | H |
| $CH_3$ | H | Br | H | $-OCF_2CHCl_2$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | Cl | H | $-OCF_2CHCl_2$ | H | H |
| $CH_3$ | $CH_3$ | Cl | H | $-OCF_2CHCl_2$ | H | Cl |
| $CH_3$ | $CH_3$ | Br | H | $-OCF_2CHCl_2$ | H | H |
| $CH_3$ | $CH_3$ | Br | H | $-OCF_2CHCl_2$ | H | Br |
| H | H | Cl | H | $-OCF_2CHF_2$ | H | H |
| H | H | Cl | H | $-OCF_2CHF_2$ | H | Cl |
| H | H | Br | H | $-OCF_2CHF_2$ | H | H |
| H | H | Br | H | $-OCF_2CHF_2$ | H | Br |
| H | $CH_3$ | Cl | H | $-OCF_2CHF_2$ | H | H |
| H | $CH_3$ | Cl | H | $-OCF_2CHF_2$ | H | Cl |
| H | $CH_3$ | Br | H | $-OCF_2CHF_2$ | H | H |
| H | $CH_3$ | Br | H | $-OCF_2CHF_2$ | H | Br |
| $CH_3$ | H | Cl | H | $-OCF_2CHF_2$ | H | H |
| $CH_3$ | H | Cl | H | $-OCF_2CHF_2$ | H | Cl |
| $CH_3$ | H | Br | H | $-OCF_2CHF_2$ | H | H |
| $CH_3$ | H | Br | H | $-OCF_2CHF_2$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $CH_3$ | Cl | H | $-OCF_2CHF_2$ | H | H |
| $CH_3$ | $CH_3$ | Cl | H | $-OCF_2CHF_2$ | H | Cl |
| $CH_3$ | $CH_3$ | Br | H | $-OCF_2CHF_2$ | H | H |
| $CH_3$ | $CH_3$ | Br | H | $-OCF_2CHF_2$ | H | Br |
| H | H | Cl | H | $-SO_2CF_3$ | H | H |
| H | H | Cl | H | $-SO_2CF_3$ | H | Cl |
| H | H | Br | H | $-SO_2CF_3$ | H | H |
| H | H | Br | H | $-SO_2CF_3$ | H | Br |
| H | H | $CF_3$ | H | $-SO_2CF_3$ | H | H |
| H | $CH_3$ | Cl | H | $-SO_2CF_3$ | H | H |
| H | $CH_3$ | Cl | H | $-SO_2CF_3$ | H | Cl |
| H | $CH_3$ | Br | H | $-SO_2CF_3$ | H | H |
| H | $CH_3$ | Br | H | $-SO_2CF_3$ | H | Br |
| H | $CH_3$ | $CF_3$ | H | $-SO_2CF_3$ | H | H |
| $CH_3$ | H | Cl | H | $-SO_2CF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | H | Cl | H | $-SO_2CF_3$ | H | Cl |
| $CH_3$ | H | Br | H | $-SO_2CF_3$ | H | H |
| $CH_3$ | H | Br | H | $-SO_2CF_3$ | H | Br |
| $CH_3$ | H | $CF_3$ | H | $-SO_2CF_3$ | H | H |
| $CH_3$ | $CH_3$ | Cl | H | $-SO_2CF_3$ | H | H |
| $CH_3$ | $CH_3$ | Cl | H | $-SO_2CF_3$ | H | Cl |
| $CH_3$ | $CH_3$ | Br | H | $-SO_2CF_3$ | H | H |
| $CH_3$ | $CH_3$ | Br | H | $-SO_2CF_3$ | H | Br |
| $CH_3$ | $CH_3$ | $CF_3$ | H | $-SO_2CF_3$ | H | H |
| H | H | F | H | $-SCCl_2F$ | H | H |
| H | H | F | H | $-SCCl_2F$ | H | F |
| H | H | F | H | $-SCCl_2F$ | F | F |
| H | H | Cl | H | $-SCCl_2F$ | H | H |
| H | H | Cl | H | $-SCCl_2F$ | H | Cl |
| H | H | Cl | Cl | $-SCCl_2F$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | Br | H | $-SCCl_2F$ | H | H |
| H | H | Br | H | $-SCCl_2F$ | H | Br |
| H | $CH_3$ | F | H | $-SCCl_2F$ | H | H |
| H | $CH_3$ | F | H | $-SCCl_2F$ | H | F |
| H | $CH_3$ | F | F | $-SCCl_2F$ | F | F |
| H | $CH_3$ | Cl | H | $-SCCl_2F$ | H | H |
| H | $CH_3$ | Cl | H | $-SCCl_2F$ | H | Cl |
| H | $CH_3$ | Cl | Cl | $-SCCl_2F$ | H | Cl |
| H | $CH_3$ | Br | H | $-SCCl_2F$ | H | H |
| H | $CH_3$ | Br | H | $-SCCl_2F$ | H | Br |
| $CH_3$ | H | F | H | $-SCCl_2F$ | H | H |
| $CH_3$ | H | F | H | $-SCCl_2F$ | H | F |
| $CH_3$ | H | F | H | $-SCCl_2F$ | F | F |
| $CH_3$ | H | Cl | H | $-SCCl_2F$ | H | H |
| $CH_3$ | H | Cl | H | $-SCCl_2F$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | H | Cl | Cl | $-SCCl_2F$ | H | Cl |
| $CH_3$ | H | Br | H | $-SCCl_2F$ | H | H |
| $CH_3$ | H | Br | H | $-SCCl_2F$ | H | Br |
| $CH_3$ | $CH_3$ | F | H | $-SCCl_2F$ | H | H |
| $CH_3$ | $CH_3$ | F | H | $-SCCl_2F$ | H | F |
| $CH_3$ | $CH_3$ | F | F | $-SCCl_2F$ | F | F |
| $CH_3$ | $CH_3$ | Cl | H | $-SCCl_2F$ | H | H |
| $CH_3$ | $CH_3$ | Cl | H | $-SCCl_2F$ | H | Cl |
| $CH_3$ | $CH_3$ | Cl | Cl | $-SCCl_2F$ | H | Cl |
| $CH_3$ | $CH_3$ | Br | H | $-SCCl_2F$ | H | H |
| $CH_3$ | $CH_3$ | Br | H | $-SCCl_2F$ | H | Br |
| H | H | F | H | $-OCHF_2$ | H | H |
| H | H | F | H | $-OCHF_2$ | H | F |
| H | H | F | F | $-OCHF_2$ | F | F |
| H | H | Cl | H | $-OCHF_2$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | H | Cl | H | $-OCHF_2$ | H | Cl |
| H | H | Cl | Cl | $-OCHF_2$ | H | Cl |
| H | H | Br | H | $-OCHF_2$ | H | H |
| H | H | Br | H | $-OCHF_2$ | H | Br |
| H | $CH_3$ | F | H | $-OCHF_2$ | H | H |
| H | $CH_3$ | F | H | $-OCHF_2$ | H | F |
| H | $CH_3$ | F | F | $-OCHF_2$ | F | F |
| H | $CH_3$ | Cl | H | $-OCHF_2$ | H | H |
| H | $CH_3$ | Cl | H | $-OCHF_2$ | H | Cl |
| H | $CH_3$ | Cl | Cl | $-OCHF_2$ | H | Cl |
| H | $CH_3$ | Br | H | $-OCHF_2$ | H | H |
| H | $CH_3$ | Br | H | $-OCHF_2$ | H | Br |
| $CH_3$ | H | F | H | $-OCHF_2$ | H | H |
| $CH_3$ | H | F | H | $-OCHF_2$ | H | F |
| $CH_3$ | H | F | F | $-OCHF_2$ | F | F |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | H | Cl | H | $-OCHF_2$ | H | H |
| $CH_3$ | H | Cl | H | $-OCHF_2$ | H | Cl |
| $CH_3$ | H | Cl | Cl | $-OCHF_2$ | H | Cl |
| $CH_3$ | H | Br | H | $-OCHF_2$ | H | H |
| $CH_3$ | H | Br | H | $-OCHF_2$ | H | Br |
| $CH_3$ | $CH_3$ | F | H | $-OCHF_2$ | H | H |
| $CH_3$ | $CH_3$ | F | H | $-OCHF_2$ | H | F |
| $CH_3$ | $CH_3$ | F | F | $-OCHF_2$ | F | F |
| $CH_3$ | $CH_3$ | Cl | H | $-OCHF_2$ | H | H |
| $CH_3$ | $CH_3$ | Cl | H | $-OCHF_2$ | H | Cl |
| $CH_3$ | $CH_3$ | Cl | Cl | $-OCHF_2$ | H | F |
| $CH_3$ | $CH_3$ | Br | H | $-OCHF_2$ | H | H |
| $CH_3$ | $CH_3$ | Br | H | $-OCHF_2$ | H | Br |

TABELLE 1 — Fortsetzung

| $R^1$ | $R^2$ | $R^3$ | $R^4$ | $R^5$ | $R^6$ | $R^7$ |
|---|---|---|---|---|---|---|
| $C_2H_5$ | H | Cl | H | $CF_3$ | H | Cl |
| $C_2H_5$ | H | Cl | Cl | $CF_3$ | H | Cl |
| $C_2H_5$ | H | Cl | H | $CF_3$ | H | H |
| $C_2H_5$ | H | Cl | H | $OCF_3$ | H | H |
| $C_2H_5$ | H | Cl | H | $SOCF_3$ | H | H |
| $C_2H_5$ | H | Cl | H | $SOCF_3$ | H | Cl |
| $C_2H_5$ | H | Cl | H | $SO_2CF_3$ | H | H |
| $C_2H_5$ | H | Cl | H | $SO_2CF_3$ | H | Cl |
| $C_2H_5$ | H | Cl | H | Br | H | Cl |
| $C_2H_5$ | $C_2H_5$ | Cl | H | $CF_3$ | H | Cl |
| $C_2H_5$ | $C_2H_5$ | Cl | Cl | $CF_3$ | H | Cl |
| $C_2H_5$ | $C_2H_5$ | Cl | H | $OCF_3$ | H | H |
| $C_2H_5$ | $C_2H_5$ | Cl | H | $OCF_3$ | H | H |
| $C_2H_5$ | $C_2H_5$ | Cl | H | $OCF_3$ | H | Cl |
| $C_2H_5$ | $C_2H_5$ | Cl | H | $SCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $C_2H_5$ | $C_2H_5$ | Cl | H | $SCF_3$ | H | Cl |
| $C_2H_5$ | $C_2H_5$ | Cl | H | $SOCF_3$ | H | H |
| $C_2H_5$ | $C_2H_5$ | Cl | H | $SOCF_3$ | H | Cl |
| $C_2H_5$ | $C_2H_5$ | Cl | H | $SO_2CF_3$ | H | H |
| $C_2H_5$ | $C_2H_5$ | Cl | H | $SO_2CF_3$ | H | Cl |
| $C_2H_5$ | $C_2H_5$ | Cl | H | Br | H | Cl |
| i-$C_3H_7$ | H | Cl | H | $CF_3$ | H | Cl |
| i-$C_3H_7$ | H | Cl | Cl | $CF_3$ | H | Cl |
| i-$C_3H_7$ | H | Cl | H | $CF_3$ | H | H |
| i-$C_3H_7$ | H | Cl | H | $OCF_3$ | H | H |
| i-$C_3H_7$ | H | Cl | H | $OCF_3$ | H | Cl |
| i-$C_3H_7$ | H | Cl | H | $SCF_3$ | H | H |
| i-$C_3H_7$ | H | Cl | H | $SCF_3$ | H | Cl |
| i-$C_3H_7$ | H | Cl | H | $SOCF_3$ | H | H |
| i-$C_3H_7$ | H | Cl | H | $SOCF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $i-C_3H_7$ | H | Cl | H | $SO_2CF_3$ | H | H |
| $i-C_3H_7$ | H | Cl | H | $SO_2CF_3$ | H | Cl |
| $i-C_3H_7$ | H | Cl | H | Br | H | Cl |
| $i-C_3H_7$ | H | Cl | H | $CF_3$ | H | Cl |
| $i-C_3H_7$ | H | Cl | Cl | $CF_3$ | H | Cl |
| $i-C_3H_7$ | H | Cl | H | $CF_3$ | H | H |
| $i-C_3H_7$ | H | Cl | H | $OCF_3$ | H | H |
| $i-C_3H_7$ | H | Cl | H | $OCF_3$ | H | Cl |
| $i-C_3H_7$ | H | Cl | H | $SCF_3$ | H | H |
| $i-C_3H_7$ | H | Cl | H | $SCF_3$ | H | Cl |
| $i-C_3H_7$ | H | Cl | H | $SOCF_3$ | H | H |
| $i-C_3H_7$ | H | Cl | H | $SOCF_3$ | H | Cl |
| $i-C_3H_7$ | H | Cl | H | $SO_2CF_3$ | H | H |
| $i-C_3H_7$ | H | Cl | H | $SO_2CF_3$ | H | Cl |
| $i-C_3H_7$ | H | Cl | H | Br | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $i-C_3H_7$ | $i-C_3H_7$ | Cl | H | $CF_3$ | H | Cl |
| $i-C_3H_7$ | $i-C_3H_7$ | Cl | Cl | $CF_3$ | H | Cl |
| $i-C_3H_7$ | $i-C_3H_7$ | Cl | H | $CF_3$ | H | H |
| $i-C_3H_7$ | $i-C_3H_7$ | Cl | H | $OCF_3$ | H | H |
| $i-C_3H_7$ | $i-C_3H_7$ | Cl | H | $OCF_3$ | H | Cl |
| $i-C_3H_7$ | $i-C_3H_7$ | Cl | H | $SCF_3$ | H | H |
| $i-C_3H_7$ | $i-C_3H_7$ | Cl | H | $SCF_3$ | H | Cl |
| $i-C_3H_7$ | $i-C_3H_7$ | Cl | H | $SOCF_3$ | H | H |
| $i-C_3H_7$ | $i-C_3H_7$ | Cl | H | $SOCF_3$ | H | Cl |
| $i-C_3H_7$ | $i-C_3H_7$ | Cl | H | $SO_2CF_3$ | H | H |
| $i-C_3H_7$ | $i-C_3H_7$ | Cl | H | $SO_2CF_3$ | H | Cl |
| $i-C_3H_7$ | $i-C_3H_7$ | Cl | H | Br | H | Cl |
| $t-C_4H_9$ | H | Cl | H | $CF_3$ | H | Cl |
| $t-C_4H_9$ | H | Cl | Cl | $CF_3$ | H | Cl |
| $t-C_4H_9$ | H | Cl | H | $CF_3$ | H | H |
| $t-C_4H_9$ | H | Cl | H | $OCF_3$ | H | H |
| $t-C_4H_9$ | H | Cl | H | $OCF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $t-C_4H_9$ | H | Cl | H | $SCF_3$ | H | H |
| $t-C_4H_9$ | H | Cl | H | $SCF_3$ | H | Cl |
| $t-C_4H_9$ | H | Cl | H | $SOCF_3$ | H | Cl |
| $t-C_4H_9$ | H | Cl | H | $SOCF_3$ | H | H |
| $t-C_4H_9$ | H | Cl | H | $SO_2CF_3$ | H | H |
| $t-C_4H_9$ | H | Cl | H | $SO_2CF_3$ | H | Cl |
| $t-C_4H_9$ | H | Cl | H | Br | H | Cl |
| H | $t-C_4H_9$ | Cl | H | $CF_3$ | H | Cl |
| H | $t-C_4H_9$ | Cl | Cl | $CF_3$ | H | Cl |
| H | $t-C_4H_9$ | Cl | H | $CF_3$ | H | H |
| H | $t-C_4H_9$ | Cl | H | $OCF_3$ | H | H |
| H | $t-C_4H_9$ | Cl | H | $OCF_3$ | H | Cl |
| H | $t-C_4H_9$ | Cl | H | $SCF_3$ | H | H |
| H | $t-C_4H_9$ | Cl | H | $SCF_3$ | H | Cl |
| H | $t-C_4H_9$ | Cl | H | $SOCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $t-C_4H_9$ | Cl | H | $SOCF_3$ | H | Cl |
| H | $t-C_4H_9$ | Cl | H | $SO_2CF_3$ | H | H |
| H | $t-C_4H_9$ | Cl | H | $SO_2CF_3$ | H | Cl |
| H | $t-C_4H_9$ | Cl | H | Br | H | Cl |
| $t-C_4H_9$ | $t-C_4H_9$ | Cl | H | $CF_3$ | H | Cl |
| $t-C_4H_9$ | $t-C_4H_9$ | Cl | Cl | $CF_3$ | H | Cl |
| $t-C_4H_9$ | $t-C_4H_9$ | Cl | H | $CF_3$ | H | H |
| $t-C_4H_9$ | $t-C_4H_9$ | Cl | H | $OCF_3$ | H | H |
| $t-C_4H_9$ | $t-C_4H_9$ | Cl | H | $OCF_3$ | H | Cl |
| $t-C_4H_9$ | $t-C_4H_9$ | Cl | H | $SCF_3$ | H | H |
| $t-C_4H_9$ | $t-C_4H_9$ | Cl | H | $SCF_3$ | H | Cl |
| $t-C_4H_9$ | $t-C_4H_9$ | Cl | H | $SOCF_3$ | H | H |
| $t-C_4H_9$ | $t-C_4H_9$ | Cl | H | $SOCF_3$ | H | Cl |
| $t-C_4H_9$ | $t-C_4H_9$ | Cl | H | $SO_2CF_3$ | H | H |
| $t-C_4H_9$ | $t-C_4H_9$ | Cl | H | $SO_2CF_3$ | H | Cl |
| $t-C_4H_9$ | $t-C_4H_9$ | Cl | H | Br | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $t-C_4H_9$ | $CH_3$ | Cl | H | $CF_3$ | H | Cl |
| $t-C_4H_9$ | $CH_3$ | Cl | Cl | $CF_3$ | H | Cl |
| $t-C_4H_9$ | $CH_3$ | Cl | H | $CF_3$ | H | H |
| $t-C_4H_9$ | $CH_3$ | Cl | H | $OCF_3$ | H | H |
| $t-C_4H_9$ | $CH_3$ | Cl | H | $OCF_3$ | H | Cl |
| $t-C_4H_9$ | $CH_3$ | Cl | H | $SCF_3$ | H | H |
| $t-C_4H_9$ | $CH_3$ | Cl | H | $SCF_3$ | H | Cl |
| $t-C_4H_9$ | $CH_3$ | Cl | H | $SOCF_3$ | H | H |
| $t-C_4H_9$ | $CH_3$ | Cl | H | $SOCF_3$ | H | Cl |
| $t-C_4H_9$ | $CH_3$ | Cl | H | $SO_2CF_3$ | H | H |
| $t-C_4H_9$ | $CH_3$ | Cl | H | $SO_2CF_3$ | H | Cl |
| $t-C_4H_9$ | $CH_3$ | Cl | H | Br | H | Cl |
| $CH_3$ | $t-C_4H_9$ | Cl | H | $CF_3$ | H | Cl |
| $CH_3$ | $t-C_4H_9$ | Cl | Cl | $CF_3$ | H | Cl |
| $CH_3$ | $t-C_4H_9$ | Cl | H | $CF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $CH_3$ | $t-C_4H_9$ | Cl | H | $OCF_3$ | H | H |
| $CH_3$ | $t-C_4H_9$ | Cl | H | $OCF_3$ | H | Cl |
| $CH_3$ | $t-C_4H_9$ | Cl | H | $SCF_3$ | H | H |
| $CH_3$ | $t-C_4H_9$ | Cl | H | $SCF_3$ | H | Cl |
| $CH_3$ | $t-C_4H_9$ | Cl | H | $SOCF_3$ | H | H |
| $CH_3$ | $t-C_4H_9$ | Cl | H | $SOCF_3$ | H | Cl |
| $CH_3$ | $t-C_4H_9$ | Cl | H | $SO_2CF_3$ | H | H |
| $CH_3$ | $t-C_4H_9$ | Cl | H | $SO_2CF_3$ | H | Cl |
| $CH_3$ | $t-C_4H_9$ | Cl | H | Br | H | Cl |
| $F_3C-$ | H | Cl | H | $CF_3$ | H | Cl |
| $F_3C-$ | H | Cl | Cl | $CF_3$ | H | Cl |
| $F_3C-$ | H | Cl | H | $CF_3$ | H | H |
| $F_3C-$ | H | Cl | H | $OCF_3$ | H | H |
| $F_3C-$ | H | Cl | H | $OCF_3$ | H | Cl |
| $F_3C-$ | H | Cl | H | $SCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $F_3C$- | H | Cl | H | $SCF_3$ | H | Cl |
| $F_3C$- | H | Cl | H | $SOCF_3$ | H | H |
| $F_3C$- | H | Cl | H | $SOCF_3$ | H | Cl |
| $F_3C$- | H | Cl | H | $SO_2CF_3$ | H | H |
| $F_3C$- | H | Cl | H | $SO_2CF_3$ | H | Cl |
| $F_3C$- | H | Cl | H | Br | H | Cl |
| H | $F_3C$- | Cl | H | $CF_3$ | H | Cl |
| H | $F_3C$- | Cl | Cl | $CF_3$ | H | Cl |
| H | $F_3C$- | Cl | H | $CF_3$ | H | H |
| H | $F_3C$- | Cl | H | $OCF_3$ | H | H |
| H | $F_3C$- | Cl | H | $OCF_3$ | H | Cl |
| H | $F_3C$- | Cl | H | $SCF_3$ | H | Cl |
| H | $F_3C$- | Cl | H | $SCF_3$ | H | H |
| H | $F_3C$- | Cl | H | $SOCF_3$ | H | H |
| H | $F_3C$- | Cl | H | $SOCF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $F_3C$- | Cl | H | $SO_2CF_3$ | H | H |
| H | $F_3C$- | Cl | H | $SO_2CF_3$ | H | Cl |
| $F_3C$- | $CH_3$ | Cl | H | $CF_3$ | H | H |
| $F_3C$- | $CH_3$ | Cl | H | $CF_3$ | H | Cl |
| $F_3C$- | $CH_3$ | Cl | H | Br | H | Cl |
| $F_3C$- | $F_3C$- | Cl | H | $CF_3$ | H | Cl |
| $F_3C$- | $F_3C$- | Cl | Cl | $CF_3$ | H | Cl |
| $F_3C$- | $F_3C$- | Cl | H | $CF_3$ | H | H |
| $F_3C$- | $F_3C$- | Cl | H | $OCF_3$ | H | H |
| $F_3C$- | $F_3C$- | Cl | H | $OCF_3$ | H | Cl |
| $F_3C$- | $F_3C$- | Cl | H | $SCF_3$ | H | H |
| $F_3C$- | $F_3C$- | Cl | H | $SCF_3$ | H | Cl |
| $F_3C$- | $F_3C$- | Cl | H | $SOCF_3$ | H | H |
| $F_3C$- | $F_3C$- | Cl | H | $SOCF_3$ | H | Cl |
| $F_3C$- | $F_3C$- | Cl | H | $SO_2CF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| $F_3C-$ | $F_3C-$ | Cl | H | $SO_2CF_3$ | H | Cl |
| $F_3C-$ | $F_3C-$ | Cl | H | Br | H | Cl |
| $H_3COCH_2-$ | H | Cl | H | $CF_3$ | H | Cl |
| $H_3COCH_2-$ | H | Cl | Cl | $CF_3$ | H | Cl |
| $H_3COCH_2-$ | H | Cl | H | $CF_3$ | H | H |
| $H_3COCH_2-$ | H | Cl | H | $OCF_3$ | H | H |
| $H_3COCH_2-$ | H | Cl | H | $OCF_3$ | H | Cl |
| $H_3COCH_2-$ | H | Cl | H | $SCF_3$ | H | H |
| $H_3COCH_2-$ | H | Cl | H | $SCF_3$ | H | Cl |
| $H_3COCH_2-$ | H | Cl | H | $SOCF_3$ | H | H |
| $H_3COCH_2-$ | H | Cl | H | $SOCF_3$ | H | Cl |
| $H_3COCH_2-$ | H | Cl | H | $SO_2CF_3$ | H | H |
| $H_3COCH_2-$ | H | Cl | H | $SO_2CF_3$ | H | Cl |
| $H_3COCH_2-$ | H | Cl | H | Br | H | H |
| H | $H_3COCH_2-$ | Cl | H | $CF_3$ | H | Cl |
| H | $H_3COCH_2-$ | Cl | Cl | $CF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $H_3COCH_2$- | Cl | H | $CF_3$ | H | H |
| H | $H_3COCH_2$- | Cl | H | $OCF_3$ | H | H |
| H | $H_3COCH_2$- | Cl | H | $OCF_3$ | H | Cl |
| H | $H_3COCH_2$- | Cl | H | $SCF_3$ | H | H |
| H | $H_3COCH_2$- | Cl | H | $SCF_3$ | H | Cl |
| H | $H_3COCH_2$- | Cl | H | $SOCF_3$ | H | H |
| H | $H_3COCH_2$- | Cl | H | $SOCF_3$ | H | Cl |
| H | $H_3COCH_2$- | Cl | H | $SO_2CF_3$ | H | H |
| H | $H_3COCH_2$- | Cl | H | $SO_2CF_3$ | H | Cl |
| H | $H_3COCH_2$- | Cl | H | Br | H | Cl |
| H | $HOCH_2$- | Cl | H | $CF_3$ | H | Cl |
| H | $HOCH_2$- | Cl | Cl | $CF_3$ | H | Cl |
| H | $HOCH_2$- | Cl | H | $CF_3$ | H | H |
| H | $HOCH_2$- | Cl | H | $OCF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| H | $HOCH_2$- | Cl | H | $OCF_3$ | H | Cl |
| H | $HOCH_2$- | Cl | H | $SCF_3$ | H | H |
| H | $HOCH_2$- | Cl | H | $SCF_3$ | H | Cl |
| H | $HOCH_2$- | Cl | H | $SOCF_3$ | H | H |
| H | $HOCH_2$- | Cl | H | $SOCF_3$ | H | Cl |
| H | $HOCH_2$- | Cl | H | $SO_2CF_3$ | H | H |
| H | $HOCH_2$- | Cl | H | $SO_2CF_3$ | H | Cl |
| H | $HOCH_2$- | Cl | H | Br | H | Cl |
| $HOCH_2$- | H | Cl | H | $CF_3$ | H | Cl |
| $HOCH_2$- | H | Cl | Cl | $CF_3$ | H | Cl |
| $HOCH_2$- | H | Cl | H | $CF_3$ | H | H |
| $HOCH_2$- | H | Cl | H | $OCF_3$ | H | H |
| $HOCH_2$- | H | Cl | H | $OCF_3$ | H | Cl |
| $HOCH_2$- | H | Cl | H | $SCF_3$ | H | H |
| $HOCH_2$- | H | Cl | H | $SCF_3$ | H | Cl |

TABELLE 1 — Fortsetzung

| $R^1$ | $R^2$ | $R^3$ | $R^4$ | $R^5$ | $R^6$ | $R^7$ |
|---|---|---|---|---|---|---|
| $HOCH_2$- | H | Cl | H | $SOCF_3$ | H | H |
| $HOCH_2$- | H | Cl | H | $SOCF_3$ | H | Cl |
| $HOCH_2$- | H | Cl | H | $SO_2CF_3$ | H | H |
| $HOCH_2$- | H | Cl | H | $SO_2CF_3$ | H | Cl |
| $HOCH_2$- | H | Cl | H | Br | H | Cl |
| 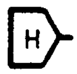 | H | Cl | H | $CF_3$ | H | Cl |
| 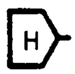 | H | Cl | Cl | $CF_3$ | H | Cl |
| 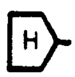 | H | Cl | H | $CF_3$ | H | H |
| 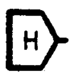 | H | Cl | H | $OCF_3$ | H | H |
| 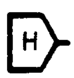 | H | Cl | H | $OCF_3$ | H | Cl |
| 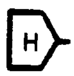 | H | Cl | H | $SCF_3$ | H | H |
| 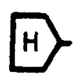 | H | Cl | H | $SCF_3$ | H | Cl |
| 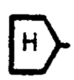 | H | Cl | H | $SOCF_3$ | H | H |
| 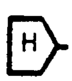 | H | Cl | H | $SOCF_3$ | H | Cl |
| 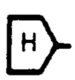 | H | Cl | H | $SO_2CF_3$ | H | H |
| 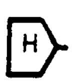 | H | Cl | H | $SO_2CF_3$ | H | H |

TABELLE 1 — Fortsetzung

| $R^1$ | $R^2$ | $R^3$ | $R^4$ | $R^5$ | $R^6$ | $R^7$ |
|---|---|---|---|---|---|---|
| 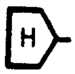 | H | Cl | H | Br | H | Cl |
| H | 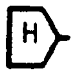 | Cl | H | $CF_3$ | H | Cl |
| H | 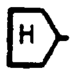 | Cl | Cl | $CF_3$ | H | Cl |
| H | 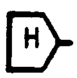 | Cl | H | $CF_3$ | H | H |
| H | 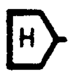 | Cl | H | $OCF_3$ | H | H |
| H | 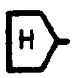 | Cl | H | $OCF_3$ | H | Cl |
| H | 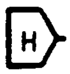 | Cl | H | $SCF_3$ | H | H |
| H | 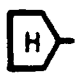 | Cl | H | $SCF_3$ | H | Cl |
| H | 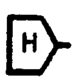 | Cl | H | $SOCF_3$ | H | H |
| H | 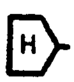 | Cl | H | $SOCF_3$ | H | Cl |
| H | 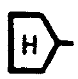 | Cl | H | $SO_2CF_3$ | H | H |
| H | 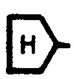 | Cl | H | $SO_2CF_3$ | H | Cl |
| H | 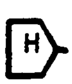 | Cl | H | Br | H | Cl |

Die erfindungsgemäß verwendbaren 4-Nitro-1-phenylpyrazole der allgemeinen Formel (I) sind teilweise bekannt (vgl. z.B. Bull. Soc. Chim. France 3727—3743 (1966); J. Chem. Soc. B, 211—214 (1968); J. Heterocycl. Chem. *7*, 345—349 (1970); Farmaco Ed. Sci. *21*, 883—891 (1966) bzw. C. A. *66*: 115640 p).

Noch nicht bekannt sind 4-Nitro-1-phenyl-pyrazole der allgemeinen Formel (Ia),

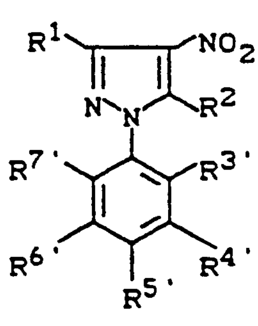

(Ia)

in welcher

$R^1$ und $R^2$ jeweils unabhängig voneinander für Wasserstoff oder für jeweils geradkettiges oder verzweigtes Alkyl, Hydroxyalkyl, Alkoxyalkyl, Alkylthioalkyl oder Halogenalkyl mit jeweils bis zu 4 Kohlenstoffatomen in den einzelnen Alkylteilen und gegebenenfalls bis zu 9 gleichen oder verschiedenen Halogenatomen oder für Cycloalkyl mit 3 bis 7 Kohlenstoffatomen stehen und

$R^{3'}$, $R^{4'}$, $R^{5'}$, $R^{6'}$ und $R^{7'}$ jeweils unabhängig voneinander für Wasserstoff, Fluor, Chlor, Brom, Jod, Cyano, Nitro, jeweils geradkettiges oder verzweigtes Alkyl, Alkoxy, Alkylthio, Alkylsulfinyl, Alkylsulfonyl oder Alkoxycarbonyl mit jeweils bis zu 4 Kohlenstoffatomen in den einzelnen Alkyltielen oder für einen Rest $-(X)_n-R^8$ stehen, wobei

X für Sauerstoff, Schwefel, Sulfinyl oder Sulfonyl steht,

n für eine Zahl 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht,

wobei jedoch mindestens einer der Reste

$R^{3'}$, $R^{4'}$, $R^{5'}$, $R^{6'}$ oder $R^{7'}$ für Cyano, für Alkylthio, Alkylsulfinyl, Alkylsulfonyl, Alkoxycarbonyl mit jeweils 1 bis 4 Kohlenstoffatomen in den einzelnen Alkylteilen oder für einen Rest $-(X)_n-R^8$ steht oder wobei mindestens drei der Reste

$R^{3'}$, $R^{4'}$, $R^{5'}$, $R^{6'}$ oder $R^{7'}$ für gleiches oder verschiedenes Halogen stehen, jedoch in dem Fall, wo $R^1$ und $R^2$ gleichzeitig für Wasserstoff stehen, mindestens einer der Reste $R^{3'}$, $R^{4'}$, $R^{5'}$, $R^{6'}$ oder $R^{7'}$ verschieden von Fluor ist.

In der allgemeinen Formel (Ia) besitzen $R^1$, $R^2$, $R^{3'}$, $R^{4'}$, $R^{5'}$, $R^{6'}$ und $R^{7'}$ allgemeinen bevorzugt diejenigen Bedeutungen, die bei der Beschreibung der Verbindungen der allgemeinen Formel (I) als bevorzugt für $R^1$, $R^2$, $R^3$, $R^4$, $R^5$, $R^6$ und $R^7$ genannt wurden, wobei jedoch mindestens einer der Reste

$R^{3'}$, $R^{4'}$, $R^{5'}$, $R^{6'}$ oder $R^{7'}$ für Cyano, für Alkylthio, Alkylsulfinyl, Alkylsulfonyl, Alkoxycarbonyl mit jeweils 1 bis 2 Kohlenstoffatomen in den einzelnen Alkylteilen oder für einen Rest $-(X)_n-R^8$ steht oder wobei mindestens drei der Reste

$R^{3'}$, $R^{4'}$, $R^{5'}$, $R^{6'}$ oder $R^{7'}$ für gleiches oder verschiedenes halogen stehen, jedoch in dem Fall, wo $R^1$ und $R^2$ gleichzeitig für Wasserstoff stehen, mindestens einer der Reste $R^{3'}$, $R^{4'}$, $R^{5'}$, $R^{6'}$ oder $R^{7'}$ verschieden von Fluor ist.

Man erhält die noch nicht bekannten 4-Nitro-1-phenylpyrazole der allgemeinen Formel (Ia), wenn man 1-Phenylpyrazole der allgemeinen Formel (II)

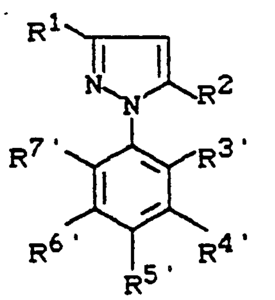

(II)

in welcher

$R^1$, $R^2$, $R^{3'}$, $R^{4'}$, $R^{5'}$, $R^{6'}$ oder $R^{7'}$ die oben angegebene Bedeutung haben,

mit Salpetersäure oder Salpetersäuresalzen, gegebenenfalls in Gegenwart eines Verdünnungsmittels und gegebenenfalls in Gegenwart eines Katalysators, nitriert.

In analoger Verfahrensweise erhält man auch die bekannten 4-Nitro-1-phenyl-pyrazole der allgemeinen Formel (I).

Verwendet man als Ausgangsstoffe beispielsweise 1-(2,6-Dichlor-4-trifluormethyl-phenyl)-pyrazol und Salpetersäure sowie Schwefelsäure als Katalysator, so läßt sich der Reaktionsablauf des Herstellungsverfahrens durch das folgende Formelschema darstellen:

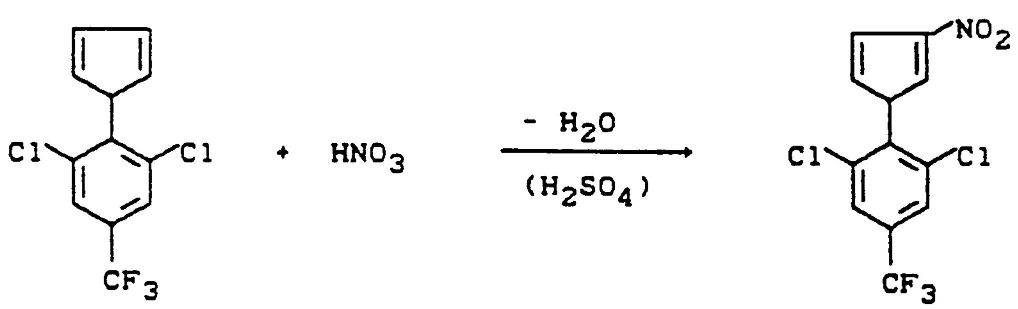

Die zur Durchführung des erfindungsgemäßen Verfahrens als Ausgangsstoffe benötigten 1-Phenylpyrazole sind durch die allgemeine Formel (II) allgemein definiert. In dieser allgemeinen Formel (II) stehen $R^1$ und $R^2$ vorzugsweise für diejenigen Reste, die schon im Zusammenhang mit der Beschreibung der erfindungsgemäß verwendbaren Stoffe der allgemeinen Formel (I) als bevorzugt für diese Substituenten genannt wurden.

Die 1-Phenylpyrazole der allgemeinen Formel (II) sind teilweise bekannt [vgl. z.B. J. Chem. Soc. Perkin Trans. *1*, 982—984 (1980); Tetrahedron Letters 925—928 (1976)].

Man erhält sie beispielsweise, wenn man 1,3-Diketone der allgemeinen Formel (III),

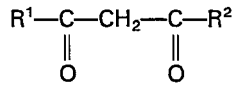 (III)

in welcher

$R^1$ und $R^2$ die oben angegebene Bedeutung haben,

oder Derivate dieser Diketone wie beispielsweise Enolether der allgemeinen Formel (IIIa),

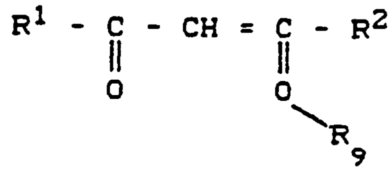 (IIIa)

in welcher

$R^1$ und $R^2$ die oben angegebene Bedeutung haben und

$R^9$ für Alkyl, Alkanoyl oder Aroyl steht,

oder ketale der allgemeinen Formeln (IIIb), (IIIc) oder (IIId)

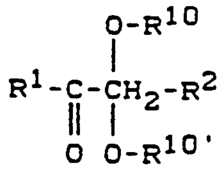
(IIIb)

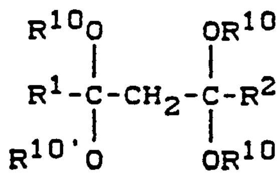
(IIIc)

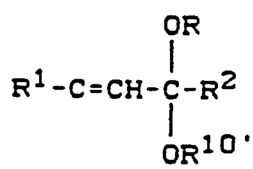
(IIId)

in welchen

$R^1$ und $R^2$ die oben angegebene Bedeutung haben,

$R^{9'}$ für Alkyl steht und

$R^{10}$ und $R^{10'}$ jeweils unabhängig voneinander für Alkyl stehen oder gemeinsam für einen zweifach verknüpften Alkandiylrest stehen,

oder Enamine der allgemeinen Formel (IIIe),

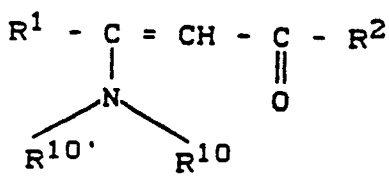 (IIIe)

in welcher

$R^1$, $R^2$, $R^{10}$ und $R^{10'}$ die oben angegebene Bedeutung haben, oder

Halogenide der allgemeinen Formel (IIIf),

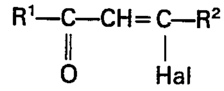 (IIIf)

in welcher

$R^1$ und $R^2$ die oben angegebene Bedeutung haben und

Hal für Halogen steht,

mit Phenylhydrazinen der allgemeinen Formel (IV),

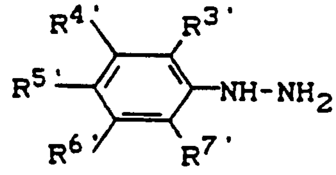 (IV)

in welcher

$R^{3'}$, $R^{4'}$, $R^{5'}$, $R^{6'}$ und $R^{7'}$ die oben angegebene Bedeutung haben,

gegenbenenfalls in Gegenwart eines Verdünnungsmittels wie beispielsweise Ethylenglykolmonoethylether oder Ethanol bei Temperaturen zwischen +50°C und +150°C cyclisiert.

Vorprodukte der allgemeinen Formel (IIa),

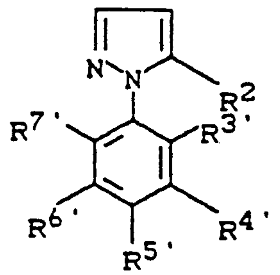

(IIa)

in welcher

$R^2$, $R^{3'}$, $R^{4'}$, $R^{5'}$, $R^{6'}$ und $R^{7'}$ die oben angegebene Bedeutung haben, erhält man alternativ auch, wenn man Phenylhydrazine der allgemeinen Formel (IV),

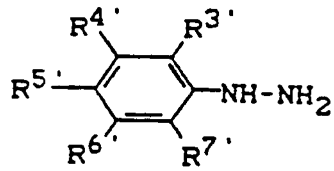

(IV)

in welcher

$R^{3'}$, $R^{4'}$, $R^{5'}$, $R^{6'}$ und $R^{7'}$ die oben angegebene Bedeutung haben,

mit Acrylesterderivaten der allgemeinen Formel (V)

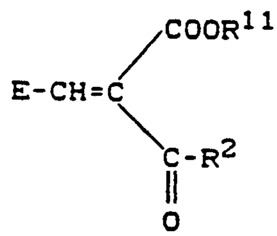

(V)

in welcher

$R^2$ die oben angegebene Bedeutung hat,

$R^{11}$ für Alkyl, insbesondere für Methyl oder Ethyl steht und

E für Alkoxy, insbesondere für Methoxy oder Ethoxy oder für Dialkylamino, insbesondere für Dimethylamino steht,

entweder zunächst in einer ersten Stufe, gegebenenfalls in Gegenwart eines Verdünnungsmittels, wie beispielsweise Ethanol oder Diethylether und gegebenenfalls in Gegenwart eines Reaktionshilfsmittels, wie beispielsweise Chlorwasserstoffsäure bei Temperaturen zwischen −20°C und +20°C umsetzt zu den Arylhydrazin-Derivaten der allgemeinen Formel (VI)

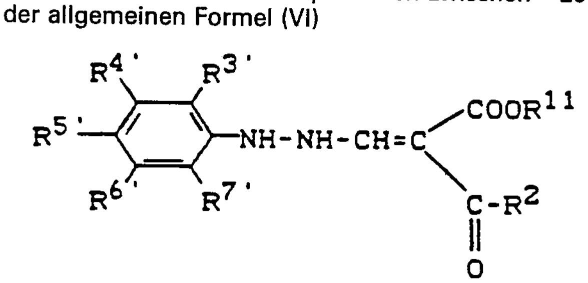

(VI)

in welcher

$R^2$, $R^{3'}$, $R^{4'}$, $R^{5'}$, $R^{6'}$, $R^{7'}$ und $R^{11}$ die oben angegebene Bedeutung haben,

und diese in einer 2. Stufe, gegebenenfalls in Gegenwart eines Verdünnungsmittels, wie beispielsweise Ethanol und gegebenenfalls in Gegenwart eines Katalysators, wie beispeilsweise Schwefelsäure bei Temperaturen zwischen +50°C und +150°C cyclisiert, oder direkt in einem Reaktionsschritt ohne Isolierung der Zwischenprodukte der allgemeinen Formel (VI) gegebenenfalls in Gegenwart eines Verdünnungsmittels wie beispielsweise Ethanol oder Ethylenglykolmonoethylether bei Temperaturen zwischen +50°C und +150°C cyclisiert;

dann die so erhaltenen 5-substituierten Pyrazol-4-carbonsäureester der allgemeinen Formel (VII),

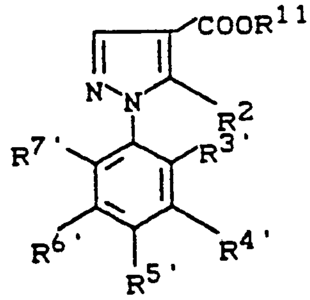

(VII)

in welcher
$R^2$, $R^{3'}$, $R^{4'}$, $R^{5'}$, $R^{6'}$, $R^{7'}$ und $R^{11}$ die oben angegebene Bedeutung haben,
in üblicher Art und Weise beispielsweise mit Basen wie Natriumhydroxid oder mit Säuren, wie Bromwasserstoffsäure gegebenenfalls in Gegenwart eines Verdünnungsmittels wie beispielsweise Ethanol oder Wasser bei Temperaturen zwischen −20°C und +100°C verseift und die so erhaltenen 5-substituierten Pyrazol-4-carbonsäuren der allgemeinen Formel (VIII)

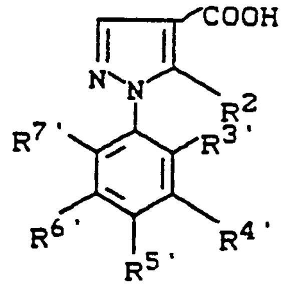

(VIII)

in welcher
$R^2$, $R^{3'}$, $R^{4'}$, $R^{5'}$, $R^{6'}$ und $R^{7'}$ die oben angegebene Bedeutung haben,
gegebenenfalls in Gegenwart eines Verdünnungsmittels wie beispielsweise Ethanol oder Wasser bei Temperaturen zwischen +100°C und +200°C decarboxyliert. Die Verseifung der Pyrazol-4-carbonsäureester der allgemeinen Formel (VII) und die anschließende Decarboxylierung der Pyrazol-4-carbonsäuren der allgemeinen formel (VIII) können dabei auch in einem Reaktionsschritt in einem sogenannten "Eintopfverfahren" ohne Isolierung der Pyrazol-4-carbonsäuren der allgemeinen Formel (VIII) durchgeführt werden.

Vorprodukte der allgemeinen Formel (IIb),

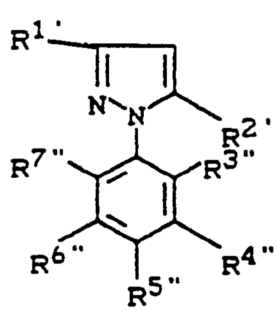

(IIb)

in welcher
$R^{1'}$ und $R^{2'}$ jeweils unabhängig voneinander für Wasserstoff oder für jeweils geradkettiges oder verzweigtes Alkyl, Hydroxyalkyl, Alkoxyalkyl oder Halogenalkyl mit jeweils bis zu 4 Kohlenstoffatomen in den einzelnen Alkylteilen und gegebenenfalls bis zu 9 gleichen oder verschiedenen halogenatomen oder für Cycloalkyl mit 3 bis 7 Kohlenstoffatomen stehen und
$R^{3''}$, $R^{4''}$, $R^{5''}$, $R^{6''}$ und $R^{7''}$ jeweils unabhängig voneinander für Wasserstoff, Fluor, Chlor, Brom, Jod, Cyano, Nitro, jeweils geradkettiges oder verzweigtes Alkyl, Alkoxy, Alkylsulfinyl, Alkylsulfonyl oder Alkoxycarbonyl mit jeweils bis zu 4 Kohlenstoffatomen in den einzelnen Alkylteilen oder für einen Rest $-(X)_n-R^8$ stehen, wobei
X' für Sauerstoff, Sulfinyl oder Sulfonyl steht,
n für eine Zahl 0 oder 1 steht und
$R^8$ für geradkettiges oder verzweigtes Halogenalkyl mit bis zu 4 Kohlenstoffatomen und bis zu 9 gleichen oder verschiedenen Halogenatomen steht,
wobei mindestens einer der Reste $R^{3''}$, $R^{4''}$, $R^{5''}$, $R^{6''}$ oder $R^{7''}$ für einen Alkylsulfinyl oder einen Alkylsulfonyl mit jeweils bis zu 4 Kohlenstoffatomen oder einen Halogenalkylsulfinyl oder einen

Halogenalkylsulfonylrest mit jeweils bis zu 4 Kohlenstoffatomen und bis zu 9 gleichen oder verschiedenen Halogenatomen steht, erhält man auch aus Verbindungen der allgemeinen Formel (IIc),

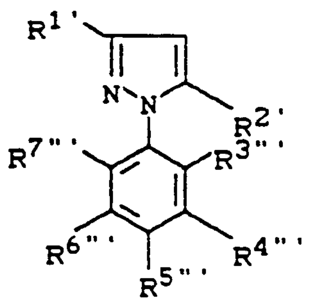

(IIc)

in welcher

$R^{1'}$ und $R^{2'}$ die oben angegebene Bedeutung haben und

$R^{3'''}$, $R^{4'''}$, $R^{5'''}$, $R^{6'''}$ und $R^{7'''}$ jeweils unabhängig voneinander für Wasserstoff, Halogen, Cyano, Nitro, jeweils geradkettiges oder verzweigtes Alkyl, Alkoxy, Alkylthio, Alkoxycarbonyl mit jeweils 1 bis 4 Kohlenstoffatomen in den einzelnen Alkylteilen oder für einen Rest $-(X'')_n-R^8$ stehen, wobei

X'' für Sauerstoff oder Schwefel steht,

n für eine Zahl 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht,

wobei mindestens einer der Reste $R^{3'''}$, $R^{4'''}$, $R^{5'''}$, $R^{6'''}$ und $R^{7'''}$ für einen Alkylthio mit bis zu 4 Kohlenstoffatomen oder einen Halogenalkylthiorest mit bis zu 4 Kohlenstoffatomen und bis zu 9 gleichen oder verschiedenen Halogenatomen steht, durch Oxidation der Alkylthio- oder Halogenalkylthiogruppe nach allgemein bekannten Verfahren mit Hilfe üblicher Oxidationsragenzien, wie beispielsweise Wasserstoffperoxid oder m-Chlorperbenzoesäure (vgl. auch Herstellungsbeispiele).

Die 1,3-Diketone der allgemeinen Formel (III) bzw. deren Derivate der allgemeinen Formeln (IIIa) bis (IIIf) und die Acrylesterderivate der allgemeinen Formel (V) sind allgemein bekannte Verbindungen der organischen Chemie [(vgl. z.B. Chem. Ber. *59,* 1282, (1926); Liebigs Ann. Chem. *452,* 182 (1927); J. Org. Chemistry *21,* 97 (1956)].

Die Phenylhydrazine der allgemeinen Formel (IV) sind größtenteils bekannt oder können nach bekannten Verfahren in einfacher, analoger Art und Weise hergestellt werden (vgl. z.B. Houben-Weyl, "Methoden der organischen Chemie" Band X/2, S. 203, Thieme Verlag Stuttgart 1967), indem man beispielsweise die bekannten Aniline der allgemeinen Formel (IX),

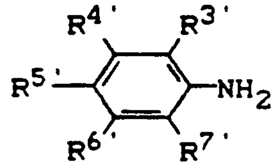

(IX)

in welcher

$R^{3'}$, $R^{4'}$, $R^{5'}$, $R^{6'}$ und $R^{7'}$ die oben angegebene Bedeutung haben,

mit Natriumnitrit in Gegenwart einer Säure, wie beispielsweise Schwefelsäure, und dann mit Zinn-IIchlorid ebenfalls in Gegenwart einer Säure, wie beispielsweise Salzsäure, bei Temperaturen zwischen −20°C und +80°C umsetzt.

Als Verdünnungsmittel zur Durchführung des Herstellungsverfahrens der noch nicht bekannten 4-Nitro-1-phenylpyrazole der allgemeinen Formel (Ia) kommen alle üblicherweise für derartige Nitrierungen einsetzbaren Lösungsmittel in Frage, Vorzugsweise verwendet man die gleichzeitig als Reagenz eingesetzte Salpetersäure oder deren Mischung mit Katalysatorsäuren, wie beispielsweise Schwefelsäure, in entsprechendem Überschuß als Verdünnungsmittel.

Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des Herstellungsverfahrens kommen ebenfalls die für derartige Nitrierungen üblichen Katalysatoren wie beispielsweise Schwefelsäure, Eisen-IIIchlorid oder andere Lewis-Säuren oder Acetanhydrid in Frage.

Die Reaktionstemperaturen können bei der Durchführung des Herstellungsverfahrens in einem größeren Bereich variiert werden. Im allgemeinen arbeitet man zwischen −50°C und +200°C, vorzugsweise zwischen −20°C und +150°C.

Zur Durchführung des Herstellungsverfahrens setzt man pro Mol 1-Phenylpyrazol der allgemeinen Formel (II) im allgemeinen 1,0 bis 30,0 Mol, vorzugsweise 1,0 bis 20,0 Mol an Salpetersäure oder

Salpetersäuresalz (vorzugsweise Kupfernitrat) und gegebenenfalls 0,1 bis 10 Mol an Katalysator oder Reaktionshilfsmittel ein. Die Reaktionsführung, Aufarbeitung und Isolierung der Reaktionsprodukte der allgemeinen Formel (Ia) erfolgt in allgemein üblicher Art und Weise.

Die erfindungsgemäß in verwendenden Wirkstoffe können als Defoliants, Desiccants, Krautabtötungsmittel und insbesondere als Unkrautvernichtungsmittel eingeseht werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäß in verwendenden Wirkstoffe können z.B. bei den folgenden Pflanzen angewandt werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycine, Beta, Daucus, Phaseolus, Pisum, Solanum, Linum, Ipomoea, vicia, Nicotiana, Lycopersicon, Arachis, Brassica, Lactuca, Cucumis, Cucurbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochloria, Fimbristylis, Sagittaria, Eleocharis, Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäß in verwendenden Wirkstoffe der allgemeinen Formel (I) neben einer besonders guten allgemeinen herbiziden Wirkung auch eine deutlich verbesserte Kulturpflanzenselektivität und lassen sich daher mit besonders gutem Erfolg zur selektiven Bekämpfung mono- und dikotyler Unkräuter in mono- und dikotylen Kulturen, wie beispielsweise Baumwolle, Soja und Getriede, einsetzen.

In entsprechenden Aufwandmengen besitzen die erfindungsgemäßen Wirkstoffe der allgemeinen Formel (I) auch eine fungizide Wirksamkeit und lassen sich beispielsweise zur Bekämpfung der Reisfleckenkrankheit (Pyricularia oryzae) einsetzen.

Darüberhinaus eignen sich die erfindungsgemäß verwendenden Wirkstoffe bei günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spex.

Aus der Ordnung der Symphyla z.B. Scutigerella immaculata.

Aus der Ordnung der Thysanura z.B. Lepisma saccharina.

Aus der Ordnung der Collembola z.B. Onychiurus aramatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratoriodes, Melanoplus differntialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis Atomaria spp., Oryzaephilus surinamensis, Anthonomus spp., Sitophilus spp., Otiorrhynhcus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp.,

Tenebrio molitor, Agroites spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum Hyalopterus, arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Haliothis spp., Laphygma exigua, Mamestra brassicae, Panolis flammea, Prodenia litura, Spodoptera spp., Trichoplusia ni, Carpocapsa pomonella, Pieris spp., Chils spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Die erfindungsgemäß in verwendenden Wirkstoffe der allgemeinen Formel (I) zeichnen sich dabei durch eine sehr gute bodeninsektizide und blattinsektizide Wirksamkeit aus.

Insbesondere aber beim Einsatz gegen Hygiene- und Vorratsschädlinge lassen sich die erfindungsgemäß in verwendenden Wirkstoffe mit besondere gutem Erfolg, so beispielsweise zur Bekämpfung des gemeinen Kornkäfers (Sitophilus granarius), einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.b. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Bei der Verwendung als Herbizide können die erfindungsgemäß in verwendenden Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethyl-ethyl)-3-methylthio-1,2,4-triazin-5(4H)-on zur Unkrautbekämpfung in Sojabohnen in Frage.

Auch Mischungen mit N,N-Dimethyl-N'-(3-trifluormethylphenyl)-harnstoff, N,N-Dimethyl-N'-(3-chlor-4-methylphenyl)-harnstoff, N,N-Dimethyl-N'-(4-isopropylphenyl)-harnstoff, 4-Amino-6-t-butyl-3-ethylthio-1,2,4-triazin-5(4H)-on, 2,4-Dichlorphenoxyessigsäure, 2,4-Dichlorphenoxypropionsäure, (2-Methyl-4-chlorphenoxy)-essigsäure, (4-Chlor-2-methyl-phenoxy)-propionsäure, Chloressigsäure-N-(methoxymethyl)-2,6-diethylanilid, 2-Ethyl-6-methyl-N-(1-methyl-2-methoxyethyl)-chloracetanilid, 2,6-Dinitro-4-trifluormethyl-N,N-dipropylanilin, 2-[4-(3,5-Dichlorpyrid-2-yloxy)-phenoxy]-propionsäure-(2-benzyloxyethylester), -(trimethylsilylmethylester) oder -(2,2-diethoxyethylester), 2-[1-(Ethoxamino)-butyliden]-5-(2-ethylthiopropyl)-1,3-cyclohexandion; 2-Chlor-N-(2,6-dimethylphenyl)-N-(1H-pyrazol-1-ylmethyl)-acetamid; N-Methyl-2-(1,3-benzthiazol-2-yloxy)-acetanilid; N,N-Di-n-propylthiocarbamidsäure-S-ethylester; exo-1-Methyl-4-(1-methylethyl)-2-(2-methylphenylphenoxy)-7-oxabicyclo[2,2,1]-heptan und 2-(4-[[3-Chlor-5-(trifluormethyl)-2-pyridinyl]-oxy]-phenoxy)-propansäure bzw. -propansäureethylester, sind möglich. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die erfindungsgemäß in verwendenden Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäß in verwendenden Wirkstoffe können sowohl vor als auch nach dem Auflaufen der Pflanzen appliziert werden.

Sie können auch vor der Saat in den Boden eingearbeitet werden.

Die Angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Auch bei der Verwendung als Insektizide können die erfindungsgemäß in verwendenden Wirkstoffe in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäß in verwendenden Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Herstellung und die Verwendung der erfindungsgemäß verwendbaren Wirkstoffe geht aus den nachfolgenden Beispielen hervor.

## Herstellungsbeispiele

### Beispiel 1

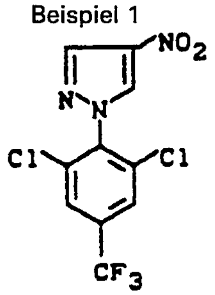

Zu 31,5 g (0.112 Mol) 1-(2,6-Dichlor-4-trifluormethylphenyl)-pyrazol in 150 ml 96-prozentiger Schwefelsäure gibt man bei Raumtemperatur 30 ml (0,714 Mol) 98-prozentige Salpetersäure so zu, daß die Temperatur der Reaktionsmischung 40°C nicht übersteigt. nach beendeter Zugabe rührt man 2 Stunden bei 50°C, kühlt dann auf ca. 15°C ab und gießt vorsichtig in ca. 1000 g Eis. Der Niederschlag wird abgesaugt, mit ca. 500 ml Wasser neutral gewashchen und bei 50°C im Vakuum getrocknet.

Man erhält 34,7 g (95% der Theorie) an 1-(2,6-Dichlor-4-trifluormethylphenyl)-4-nitropyrazol vom Schmelzpunkt 91—97°C.

Beispiel 2

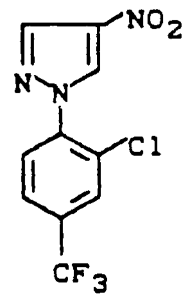

Zu einer Lösung von 6,8 ml (0,15 Mol) 98-prozentiger Salpetersäure in 22 ml Eisessig und 16 ml Acetanhydrid tropft man bei 10 bis 15°C unter Rühren eine Lösung von 6 g (0,025 Mol) 1-(2-Chlor-4-trifluormethylphenyl)-pyrazol in 22 ml Eisessig. Nach beendeter Zugabe erwärmt man die Mischung langsam auf 40°C und rührt bei dieser Temperatur 15 Stunden lang. Zur Aufarbeitung gießt man die Reaktionsmischung vorsichtig in ca. 600 ml Eiswasser, saugt den ausgefallenen Niederschlag ab, wäscht mit ca. 300 ml Wasser nach und trocknet im Vakuum bei 40 bis 50°C.

Man erhält so 6,1 g (85% der Theorie) an 1-(2-Chlor-4-trifluormethylphenyl)-4-nitro-pyrazol vom Schmelzpunkt 106—109°C.

Beispiel 3

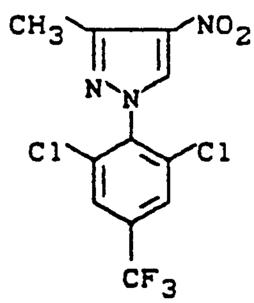

Zu einer Lösung aus 5,9 g (0,02 Mol) 1-(2,6-Dichlor-4-trifluormethylphenyl)-3-methylpyrazol in 28 ml konzentrierter Schwefelsäure werden bei 20°C langsam 5,3 ml (0,12 Mol) 98 %ige Salpetersäure getropft. Nach beendeter Zugabe wird 4 Stunden bei Raumtemperatur gerührt, auf Eiswasser gegossen und mit Methylenchlorid extrahiert. Die organische Phase wird mit gesättigter Natriumcarbonat-Lösung und Wasser gewaschen, über Magnesiumsulfat getrocknet und das Lösungsmittel im Vakuum abdestilliert.

Man erhält 5 g (73% der Theorie) an 1-(2,6-Dichlor-4-trifluormethylphenyl)-3-methyl-4-nitropyrazol vom Schmelzpunkt 76—82°C (das ca. 6% des 5-Methylisomeren enthält).

Beispiel 4

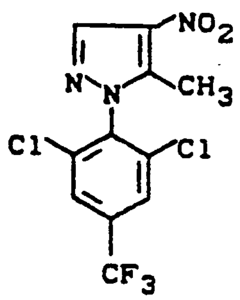

5 g (0,0147 Mol) 4-Carboxy-5-methyl-1-(2,6-dichlor-4-trifluormethyl-phenyl)-pyrazol werden in 36 ml konzentrierter Salzsäure gelöst. Man tropft 3,8 ml (0.0394 Mol) 98%ige Salpetersäure zu, erhitzt 1 Stunde auf 120°C und läßt dann abkühlen. Die Reaktionsmischung wird auf Eiswasser gegeben, mit Methylenchlorid extrahiert, die Methylenchloridphasen mit gesättigter Natriumhydrogencarbonatlösung gewaschen, über Magnesiumsulfat getrocknet und im Vakuum eingeengt.

Man erhält 3,7 g (74% der Theorie) an 5-Methyl-4-nitro-1-(2,6-dichlor-4-trifluormethyl-phenyl)-pyrazol vom Schmelzpunkt 91—92°C.

In entsprechender Weise und gemäß den allgemeinen Angaben zur Herstellung erhält man die folgenden 4-Nitro-1-phenyl-pyrazole der allgemeinen Formel (I):

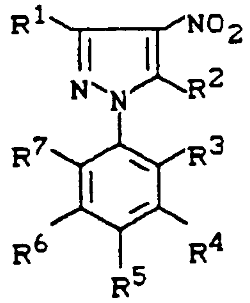

(I)

Tabelle 2

| Bsp. Nr. | $R^1$ | $R^2$ | $R^3$ | $R^4$ | $R^5$ | $R^6$ | $R^7$ | Physikal. Eigenschaften Schmelzpunkt /° C |
|---|---|---|---|---|---|---|---|---|
| 5 | H | H | Cl | H | $F_3CS$- | H | Cl | 89 |
| 6 | H | H | Cl | H | $F_3C$-SO- | H | Cl | 140 |
| 7 | H | H | Cl | Cl | $F_3C$- | H | Cl | 92-94 |
| 8 | H | H | Cl | H | $F_3C-SO_2$- | H | Cl | 218-219 |
| 9 | H | H | Cl | H | $F_3C-SO_2$- | H | H | 84-85 |
| 10 | H | H | Cl | $NO_2$ | $F_3CS$- | H | Cl | 155-159 |
| 11 | H | H | Cl | $NO_2$ | $F_3C$- | H | Cl | 115-123 |
| 12 | H | H | Cl | Cl | Cl | H | H | 135-137 |
| 13 | $CH_3$ | $CH_3$ | Cl | H | $F_3C$- | H | Cl | 83 |
| 14 | H | H | H | H | $NO_2$ | H | H | 118-120 |
| 15 | H | H | Br | H | $F_3C$- | H | Br | 120-121 |
| 16 | H | H | Cl | $NO_2$ | H | H | Cl | 147-151 |
| 17 | H | H | F | F | $F_3C$- | F | F | 70- 72 |
| 18 | H | H | Cl | F | $F_3C$- | F | Cl | 89- 90 |
| 19 | H | $CH_3$ | Cl | H | $F_3C$- | H | H | $^1$HNMR*:8,27 |
| 20 | H | n-$C_3H_7$ | Cl | H | $F_3C$- | H | Cl | 64- 70 |
| 21 | H | n-$C_3H_7$ | Cl | Cl | $F_3C$- | H | Cl | $^1$H-NMR*:8,38 |
| 22 | t-$C_4H_9$ | H | Cl | H | $F_3C$- | H | Cl | 65- 68 |
| 23 | H | $CH_3$ | Cl | $NO_2$ | Cl | H | Cl | 110 |
| 24 | H | n-$C_3H_7$ | Cl | $NO_2$ | Cl | H | Cl | 147 |

* Die $^1$H-NMR-Sprekten wurden in $CDCl_3$ mit Trimethylsilan als inneren Standard aufgenommen. Angegeben ist die chemische Verschiebung als δ-Wert in ppm.

Herstellung der Ausgangsverbindungen

Beispiel II-1

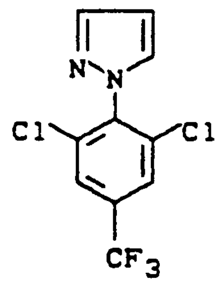

Zu 24,3 g (0,1 Mol) 2,6-Dichlor-4-trifluormethylphenylhydrazin in 100 ml Ethanol gibt man nacheinander 16,4 g (0,1 Mol) 1,1,3,3-Tetramethoxypropan und 5,1 g (0,05 Mol) 96-prozentige Schwefelsäure und erhitzt für 2 Stunden auf Rückflußtemperatur. Nach dem Abkühlen der Reaktionsmischung neutralisiert man mit 5,3 g (0,05 Mol) Natriumcarbonat, rührt eine Stunde bei Raumtemperatur und engt dann im Vakuum ein. Der Rückstand wird in 250 ml Wasser aufgenommen und mit 200 ml Methylenchlorid extrahiert. Die organische Phase wird mit Wasser gewaschen, über Natriumsulfat getrocknet und im Vakuum vom Lösungsmittel befreit.

Man erhält 23,9 g (86,5% der Theorie) an 1-(2,6-Dichlor-4-trifluormethylphenyl)-pyrazol vom Schmelzpunkt 83—85°C.

Beispiel II-2

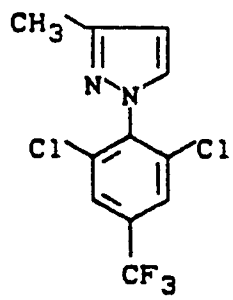

14 g (0,05 Mol) 2,6-Dichlor-4-trifluormethylphenylhydrazin und 6,6 g (0,05 Mol) 3-oxobutyraldehyddimethylacetal werden in 100 ml Ethanol gelöst und über Nacht bei Rückflußtemperatur gerührt. Nach Abkühlen auf Raumtemperatur gibt man 1 ml konzentrierte Schwefelsäure zu, rührt ca. 5 Stunden bei 60°C und engt dann im Vakuum ein. Der Rückstand wird in Methylenchlorid aufgenommen, mit gesättigter Natriumcarbonat-Lösung und Wasser gewashchen, über Magnesiumsulfat getrocknet und das Lösungsmittel im Vakuum abdestilliert.

Nach Destillation des Rückstandes bei 100°C/0,06 mbar erhält man 10 g (68% der Theorie) an 1-(2,6-Dichlor-4-trifluormethylphenyl)-3-methylpyrazol, das ca. 6% des 5-Methylisomeren enthält.

Beispiel II-3

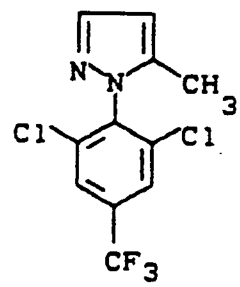

3,7 g (0,01 Mol) 4-Ethoxycarbonyl-5-methyl-1-(2,6-dichlor-4-tri-fluormethyl-phenyl)-pyrazol werden in 40 ml Bromwasserstoffsäure 48 Stunden auf 120—125°C erhitzt, wobei das entstehende Ethanol gleichzeitig abdestilliert wird. Anschließend wird die überschüssige Bromwasserstoffsäure abdestilliert, der Rückstand in Methylenchlorid aufgenommen und mit gesättigter Natriumhydrogencarbonatlösung gewaschen. Die organische Phase wird über Magnesiumsulfat getrocknet und im Vakuum eingeengt.

Man erhält 2,0 g (67% der Theorie) an 5-Methyl-1-(2,6-dichlor-4-trifluormethyl-phenyl)-pyrazol vom Schmelzpunkt 78—84°C.

Beispiel II-4

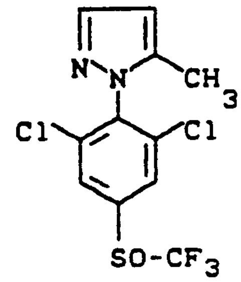

In 51,7 g (0,165 Mol) 1-(2,6-Dichlor-4-trifluormethylthiophenyl)-pyrazol in 300 ml Dichlormethan gibt man 34,8 g (0,182 Mol) 90-prozentige m-Chlorperbenzoesäure und rührt 18 Stunden bei Raumtemperatur. Zur Aufarbeitung wird die Reaktionsmischung filtriert, das Filtrat nacheinander mit wäßriger Natriumhydrogencarbonat-, Natriumthiosulfat- und Natriumchloridlösung gewaschen und über eine Kieselgelsäule (Laufmittel: Dichlormethan) chromatographisch gereinigt. Man erhält 20,6 g (54% der Theorie) an 1-(2,6-Dichlor-4-trifluormethylsulfinylphenyl)-pyrazol vom Schmelzpunkt 86—91°C (neben 15,6 g unumgesetzter Ausgangsverbindung).

In entsprechender Weise und gemäß den allgemeinen Angaben der Herstellung erhält man die folgenden 1-Phenylpyrazole der allgemeinen Formel (II):

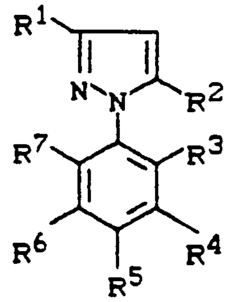

(II)

Tabelle 3

| Bsp. Nr. | $R^1$ | $R^2$ | $R^3$ | $R^4$ | $R^5$ | $R^6$ | $R^7$ | Physikal. Eigenschaften Schmelzpunkt/° C |
|---|---|---|---|---|---|---|---|---|
| II-5 | H | H | Cl | Cl | $F_3C$- | H | Cl | 49-52 |
| II-6 | H | H | Cl | H | $F_3C$- | H | H | $^1$H-NMR*: |
| | | | | | | Pyrazol | | H-3: 7,97 |
| | | | | | | Pyrazol | | H-4: 6,46 |
| II-7 | H | H | Cl | H | $F_3C-SO_2$- | H | H | 69-70 |
| II-8 | H | H | Cl | H | $F_3C-SO_2$- | H | Cl | 113-115 |
| II-9 | H | H | Cl | H | $F_3CS$- | H | Cl | $^1$H-NMR*: |
| | | | | | | Pyrazol | | H-3: 7,81 |
| | | | | | | Pyrazol | | H-4: 6,51 |
| II-10 | $CH_3$ | $CH_3$ | Cl | H | $F_3C$- | H | Cl | 80-81 |
| II-11 | H | $CH_3$ | Cl | H | $F_3C$- | H | H | 45-47 |
| II-12 | H | $CH_3$ | Cl | H | $F_3C-SO_2$- | H | H | 99-100 |
| II-13 | H | n-$C_3H_7$ | Cl | H | $F_3C-SO_2$- | H | H | 66-67 |
| II-14 | H | $CF_3$ | $NO_2$ | H | $NO_2$ | H | H | 166 |
| II-15 | H | H | Br | H | $F_3C$- | H | Br | 116-120 |

* Die $^1$H-NMR-Spektren wurden in $CDCl_3$ mit Trimethylsilan als innerem Standard aufgenommen. Angegeben ist die chemische Verschiebung als δ-Wert in ppm.

Beispiel VIII-1:

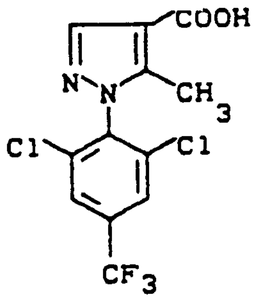

8,7 g (0,0237 Mol) 4-Ethoxycarbonyl-5-methyl-1-(2,6-dichlor-4-trifluormethyl-phenyl)-pyrazol werden in einer Lösung aus 50 ml Ethanol, 1,5 g (0,0375 Mol) Natriumhydroxid und 25 ml Wasser gelöst und 48 Stunden bei Raumtemperatur gerührt. Die Reaktionsmischung wird mit Methylenchlorid gewaschen und die alkalische Phase unter Eiskühlung mit 2N Salzsäure angesäuert. Anschließend wird mit Methylenchlorid extrahiert, die Methylenchloridphase über Magnesiumsulfat getrocknet und im Vakuum eingeengt.

Man erhält 8 g (100% der Theorie) an 4-Carboxy-5-methyl-1-(2,6-dichlor-4-trifluormethyl-phenyl)-pyrazol vom Schmelzpunkt 187—189°C.

In entsprechender Weise erhält man:

Beispiel VIII-2:

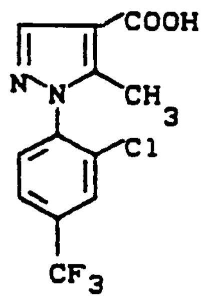

Schmelzpunkt: 161 —162° C

Beispiel VIII-3:

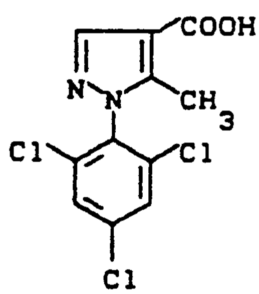

Schmelzpunkt 168-173° C

Beispiel VIII-4:

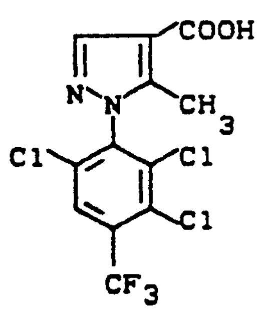

175-180° C

Beispiel VIII-5:

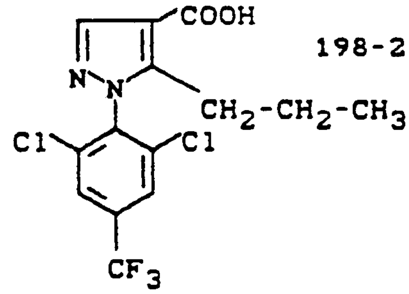

198-200° C

Beispiel VIII-6:

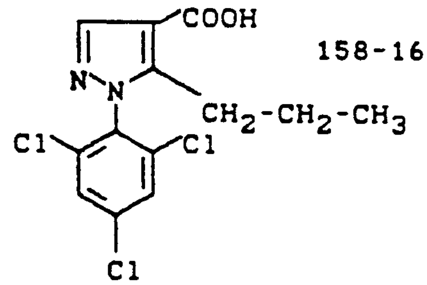

158-161° C

Beispiel VIII-7:

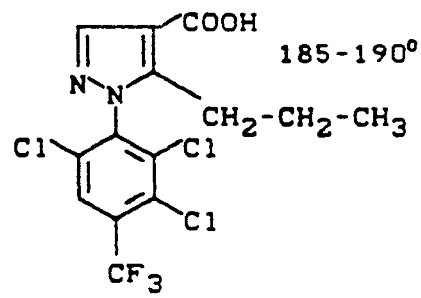

185-190° C

Beispiel VII-1:

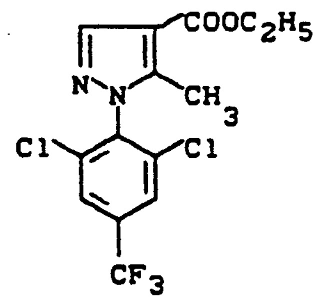

5 g (0.013 Mol) N-[2-Ethoxycarbonyl-2-acetyl-vinyl]-N'-(2,6-dichlor-4-trifluormethyl-phenyl)-hydrazin werden in 50 ml Ethanol gelöst, mit 1 ml konzentrierter Schewefelsäure versetzt und 1 Stunde unter Rückfluß erhitzt. Die Reaktionsmischung wird im Vakuum eingeengt, in Methylenchlorid aufgenommen, mit gesättigter Natriumhydrogencarbonatlösung gewaschen, die Methylenchloridphase über Magnesiumsulfat getrocknet und im Vakuum eingeengt.

Man erhält 4,1 g (86% der Theorie) an 4-Ethoxycarbonyl-5-methyl-1-(2,6-dichlor-4-trifluormethyl-phenyl)-pyrazol vom Schmelzpunkt 61—62°C.

In entsprechender Weise erhält man

Beispiel VII-2:

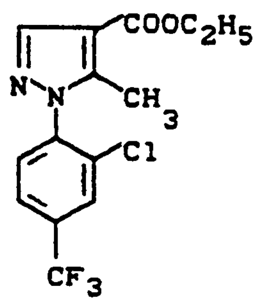

Schmelzpunkt

69 –70° C

Beispiel VII-3:

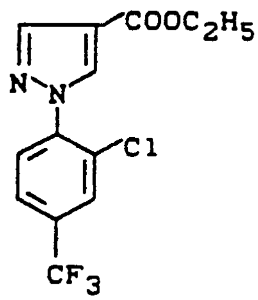

Schmelzpunkt

160 -161° C

Beispiel VII-4:

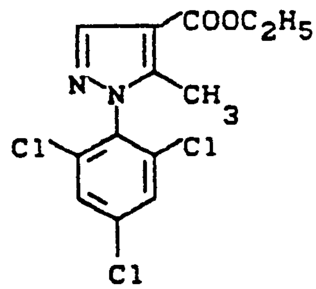

101 -104° C

Beispiel VII-5:

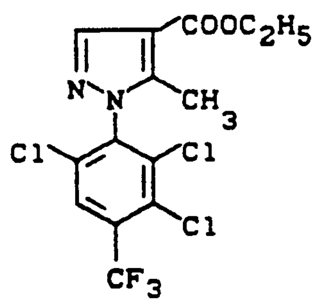

$^{1}$H-NMR: 8,17

Beispiel VII-6:

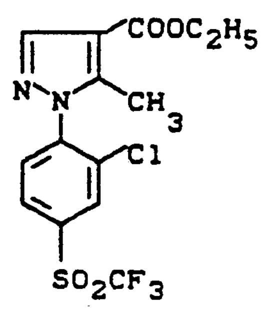

124 -125° C

Beispiel VII-7:

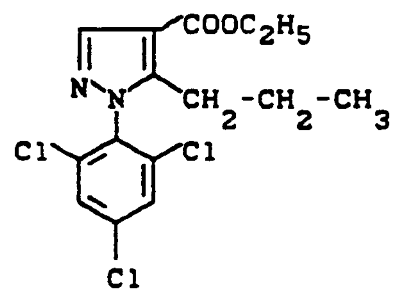

Schmelzpunkt

42-46° C

Beispiel VII-8:

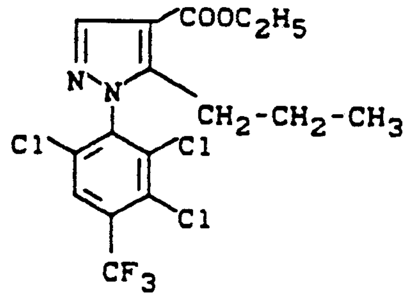

58-60° C

Beispiel VII-9:

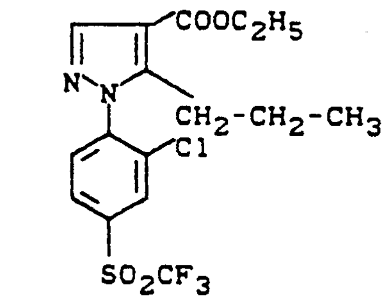

104 -105° C

Beispiel VII-10:

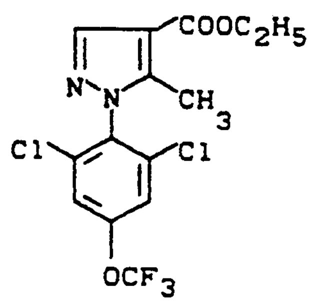

55-65° C

Beispiel VII-11:

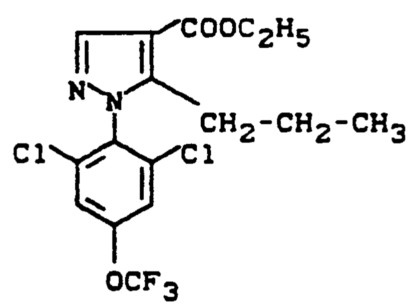

$^1$H-NMR: 8,15

Beispiel VII-12:

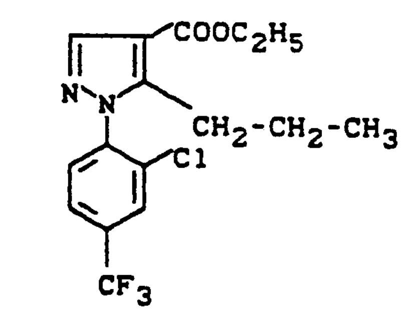

Schmelzpunkt

59-64° C

Beispiel VI-1:

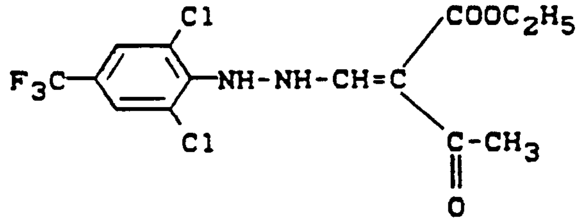

Zu einer Lösung aus 9,3 g (0,5 Mol) Ethoxymethylenacetessigsäurethylester in 50 ml Diethylether gibt man bei 0°C tropfenweise unter Rühren 12,25 g (0,05 Mol) 2,6-Dichlor-4-trifluormethylphenylhydrazin in 25 ml Diethylether. Nach beendeter Zugabe rührt man eine weitere Stunde bei Raumtemperatur und engt dann im Vakuum ein.

Man erhält 19 g (100% der Theorie) an N-[2-Ethoxycarbonyl-2-acetyl-vinyl]-N'-(2,6-dichlor-4-(trifluormethyl-phenyl)-hydrazin vom Schmelzpunkt 75—76°C.

## Anwendungsbeispiele

In den folgenden Anwendungsbeispielen wurden die nachstehend aufgeführten Verbindungen als Vergleichsverbindungen eingesetzt:

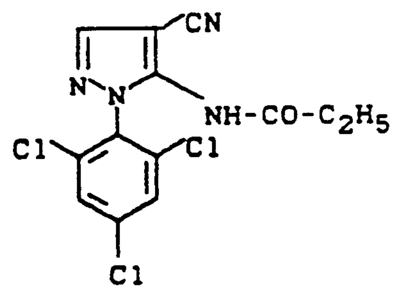

(A)

4-Cyano-5-propionylamino-1-(2,4-6-trichlorphenyl)-pyrazol (bekannt aus DE—OS 3 226 513)

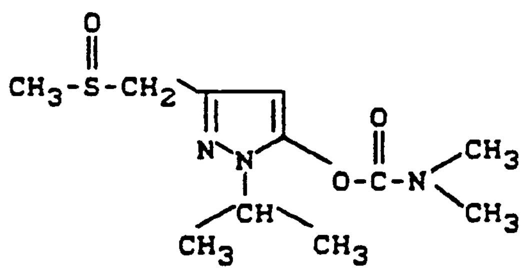

(B)

5-Dimethylaminocarbonyloxy-1-isopropyl-3-methylsulfinylmethylpyrazol (bekannt aus DE—OS 2 819 932)

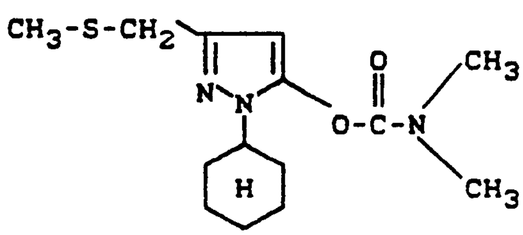

(C)

1-Cyclohexyl-5-dimethylaminocarbonyloxy-3-methylthiomethylpyrazol (bekannt aus DE—OS 2 839 270).

### Beispiel A

Pre-emergence Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen: (1) und (7).

Beispiel B

$LD_{100}$-Test

Testtiere: Sitophilus granarius

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigt z.B. die folgende Verbindung der Herstellungsbeispiele überlegene Wirkung gegenüber dem Stand der Technik: (1).

**Patentansprüche**

1. Herbizide und insektizide Mittel, gekennzeichnet durch einen Gehalt an mindestens einem 4-Nitro-1-phenyl-pyrazol der allgemeinen Formel (I),

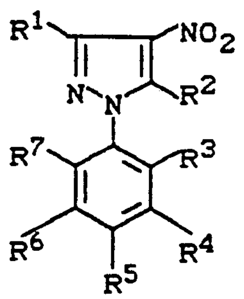

(I)

in welcher

$R^1$ und $R^2$ jeweils unabhängig voneinander für Wasserstoff oder für jeweils geradkettiges oder verzweigtes Alkyl, Hydroxyalkyl, Alkoxyalkyl, Alkylthioalkyl oder Halogenalkyl mit jeweils bis zu 4 Kohlenstoffatomen in den einzelnen Alkylteilen und gegebenenfalls bis zu 9 gleichen oder verschiedenen Halogenatomen oder für Cycloalkyl mit 3 bis 7 Kohlenstoffatomen stehen und

$R^3$, $R^4$, $R^5$, $R^6$ und $R^7$ jeweils unabhängig voneinander für Wasserstoff, Fluor, Chlor, Brom, Jod, Cyano, Nitro, jeweils geradkettiges oder verzweigtes Alkyl, Alkoxy, Alkylthio, Alkylsulfinyl, Alkylsulfonyl oder Alkoxycarbonyl mit jeweils bis zu 4 Kohlenstoffatomen in den einzelnen Alkylteilen oder für einen Rest $-(X)_n-R^8$ stehen, wobei

X für Sauerstoff, Schwefel, Sulfinyl oder Sulfonyl steht,

n für eine Zahl 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht.

2. Herbizide und insektizide Mittel, gekennzeichnet durch einen Gehalt an mindestens einem 4-Nitro-1-phenyl-pyrazol der allgemeinen Formel (I) gemäß Anspruch 1, in welcher

$R^1$ und $R^2$ unabhängig voneinander für Wasserstoff, Methyl, Ethyl, n- oder i-Propyl, n-, i-, s-, oder t-Butyl, Cyclohexyl, Hydroxymethyl, Methoxymethyl, Methylthiomethyl, Trifluormethyl oder Trichlormethyl stehen und

$R^3$, $R^4$, $R^5$, $R^6$ und $R^7$ jeweils unabhängig voneinander für Wasserstoff, Fluor, Chlor, Brom, Jod, Cyano, Nitro, Methyl, Ethyl, n- und i-Propyl, Methoxy, Ethoxy, Methylthio, Methylsulfinyl, Methylsulfonyl, Methoxycarbonyl, Ethoxycarbonyl oder für einen Rest $-(X)_n-R^8$ stehen, wobei

X für Sauerstoff, Schwefel, Sulfinyl oder Sulfonyl steht,

n für 0 oder 1 steht und

$R^8$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl steht.

3. Verfahren zur Bekämpfung von Unkraut und tierischen Schädlingen, dadurch gekennzeichnet, daß man 4-Nitro-1-phenyl-pyrazole der allgemeinen Formel (I) gemäß Anspruch 1 auf Unkraut und/oder tierische Schädlinge und/oder deren Lebensraum einwirken läßt, wobei die vom Gesetz gemäß Artikel 52 (4) EPÜ nicht zulässigen Verfahren ausgenommen sind.

4. Verwendung von 4-Nitro-1-phenyl-pyrazolen der allgemeinen Formel (I) gemäß Anspruch 1 zur Bekämpfung von Unkraut und/oder tierischen Schädlingen, wobei die Anwendung in der Human- oder Veterinärmedizin ausgenommen ist.

5. Verfahren zur Herstellung von herbiziden und insektiziden Mitteln, dadurch gekennzeichnet, daß man 4-Nitro-1-phenyl-pyrazole der allgemeinen Formel (I) gemäß Anspruch 1 mit Streckmitteln und/oder oberflächenaktiven Mitteln vermischt.

6. 4-Nitro-1-phenyl-pyrazole der allgemeinen Formel (Ia),

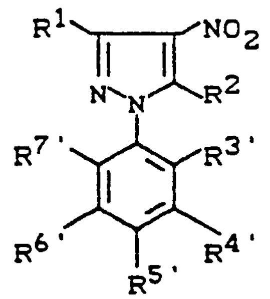

(Ia)

in welcher

$R^1$ und $R^2$ jeweils unabhängig voneinander für Wasserstoff oder für jeweils geradkettiges oder verzweigtes Alkyl, Hydroxyalkyl, Alkoxyalkyl, Alkylthioalkyl oder Halogenalkyl mit jeweils bis zu 4 Kohlenstoffatomen in den einzelnen Alkylteilen und gegebenenfalls bis zu 9 gleichen oder verschiedenen Halogenatomen oder für Cycloalkyl mit 3 bis 7 Kohlenstoffatomen stehen und

$R^{3'}$, $R^{4'}$, $R^{5'}$, $R^{6'}$ und $R^{7'}$ jeweils unabhängig voneinander für Wasserstoff, Fluor, Chlor, Brom, Jod, Cyano, Nitro, jeweils geradkettiges oder verzweigtes Alkyl, Alkoxy, Alkylthio, Alkylsulfinyl, Alkylsulfonyl oder Alkoxycarbonyl mit jeweils bis zu 4 Kohlenstoffatomen in den einzelnen Alkylteilen oder für einen Rest $-(X)_n-R^8$ stehen, wobei

X für Sauerstoff, Schwefel, Sulfinyl oder Sulfonyl steht,

n für eine Zahl 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht.

wobei jedoch mindestens einer der Reste

$R^{3'}$, $R^{4'}$, $R^{5'}$, $R^{6'}$ oder $R^{7'}$ für Cyano, für Alkylthio, Alkylsulfinyl, Alkylsulfonyl, Alkoxycarbonyl mit jeweils 1 bis 4 Kohlenstoffatomen in den einzelnen Alkylteilen oder für einen Rest $-(X)_n-R^8$ steht oder wobei mindestens drei der Reste

$R^{3'}$, $R^{4'}$, $R^{5'}$, $R^{6'}$ oder $R^{7'}$ für gleiches oder verschiedenes Halogen stehen, jedoch in dem Fall, wo $R^1$ und $R^2$ gleichzeitig für Wasserstoff stehen, mindestens einer der Reste $R^{3'}$, $R^{4'}$, $R^{5'}$, $R^{6'}$ oder $R^{7'}$ verschieden von Fluor ist.

7. Verfahren zur Herstellung von 4-Nitro-1-phenyl-pyrazolen der allgemeinen Formel (Ia),

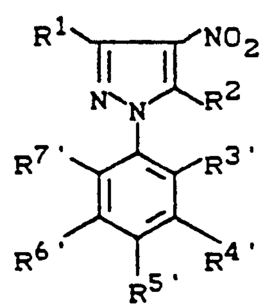

(Ia)

in welcher

$R^1$ und $R^2$ jeweils unabhängig voneinander für Wasserstoff oder für jeweils geradkettiges oder verzweigtes Alkyl, Hydroxyalkyl, Alkoxyalkyl, Alkylthioalkyl oder Halogenalkyl mit jeweils bis zu 4 Kohlenstoffatomen in den einzelnen Alkylteilen und gegebenenfalls bis zu 9 gleichen oder verschiedenen Halogenatomen oder für Cycloalkyl mit 3 bis 7 Kohlenstoffatomen stehen und

$R^{3'}$, $R^{4'}$, $R^{5'}$, $R^{6'}$ und $R^{7'}$ jeweils unabhängig voneinander für Wasserstoff, Fluor, Chlor, Brom, Jod, Cyano, Nitro, jeweils geradkettiges oder verzweigtes Alkyl, Alkoxy, Alkylthio, Alkylsulfinyl, Alkylsulfonyl oder Alkoxycarbonyl mit jeweils bis zu 4 Kohlenstoffatomen in den einzelnen Alkylteilen oder für einen Rest $-(X)_n-R^8$ stehen, wobei

X für Sauerstoff, Schwefel, Sulfinyl oder Sulfonyl steht,

n für eine Zahl 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht,

wobei jedoch mindestens drei der Reste

$R^{3'}$, $R^{4'}$, $R^{5'}$, $R^{6'}$ oder $R^{7'}$ für Cyano, für Alkylthio, Alkylsulfinyl, Alkylsulfonyl, Alkoxycarbonyl mit jeweils 1 bis 4 Kohlenstoffatomen in den einzelnen Alkylteilen oder für einen Rest $-(X)_n-R^8$ steht oder wobei mindestens drei der Reste

$R^{3'}$, $R^{4'}$, $R^{5'}$, $R^{6'}$ oder $R^{7'}$ für gleiches oder verschiedenes Halogen stehen, jedoch in dem Fall, wo $R^1$ und $R^2$ gleichzeitig für Wasserstoff stehen, mindestens einer der Reste $R^{3'}$, $R^{4'}$, $R^{5'}$, $R^{6'}$ oder $R^{7'}$ verschieden von Fluor ist, dadurch gekennzeichnet, daß man 1-Phenylpyrazole der allgemeinen Formel (II),

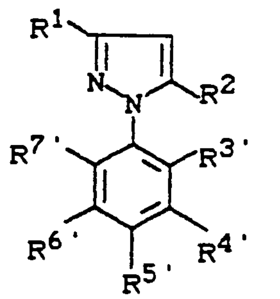

(II)

in welcher

$R^1$, $R^2$, $R^{3'}$, $R^{4'}$, $R^{5'}$, $R^{6'}$ und $R^{7'}$ die oben angegebene Bedeutung haben, mit Salpetersäure oder Salpetersäuresalzen, gegebenenfalls in Gegenwart eines Verdünnungsmittels und gegebenenfalls in Gegenwart eines Katalysators nitriert.

**Revendications**

1. Agents herbicides et insecticides caractérisés en ce qu'ils contiennent au moins un 4-nitro-1-phényl-pyrazole de formule générale (I):

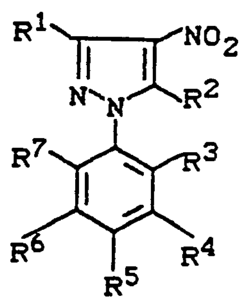

(I)

dans laquelle

$R^1$ et $R^2$ représentent chacun indépendamment l'un de l'autre un atome d'hydrogène ou un groupe halogénalkyle, un groupe alkylthioalkyle, un groupe alcoxyalkyle, un groupe hydroxyalkyle ou un groupe alkyle chaque fois à chaîne droite ou ramifiée et contenant chacun jusqu'à 4 atomes de carbone dans les fractions alkyle individuelles, et éventuellement jusqu'à 9 atomes d'halogènes identiques ou différents, ou un groupe cycloalkyle contenant 3 à 7 atomes de carbone, et

$R^3$, $R^4$, $R^5$, $R^6$ et $R^7$ représentant chacun indépendamment l'un de l'autre un atome d'hydrogène, un atome de fluor, un atome de chlore, un atome de brome, un atome d'iode, un groupe cyano, un groupe nitro, un groupe alcoxycarbonyle, un groupe alkylsulfonyle, un groupe alkylsulfinyle, un groupe alkylthio, un groupe alcoxy ou un groupe alkyle chaque fois à chaîne droite ou ramifiée contenant chacun jusqu'à 4 atomes de carbone dans les fractions alkyle individuelles, ou un radical $-(X)_n-R^8$,

X représentant un atome d'oxygène, un atome de soufre, un groupe sulfinyle ou un groupe sulfonyle, n représentant un nombre de 0 ou 1, et

$R^8$ représentant un groupe halogénalkyle à chaîne droite ou ramifiée contenant jusqu'à 4 atomes de carbone et jusqu'à 9 atomes d'halogènes identiques ou différents.

2. Agents herbicides et insecticides, caractérisés en ce qu'ils contiennent au moins un 4-nitro-1-phényl-pyrazole de formule générale (I) selon la revendication 1, dans laquelle

$R^1$ et $R^2$ représentent chacun indépendamment l'un de l'autre un atome d'hydrogène, un groupe méthyle, un groupe éthyle, un groupe n-propyle, un groupe i-propyle, un group n-butyle, un groupe i-butyle, un groupe s-butyle, un groupe t-butyle, un groupe cyclohexyle, un groupe hydroxyméthyle, un groupe méthoxyméthyle, un groupe méthylthiométhyle, un groupe trifluorométhyle ou un groupe trichlorométhyle, et

$R^3$, $R^4$, $R^5$, $R^6$ et $R^7$ représentant chacun indépendamment l'un de l'autre un atome d'hydrogène, un atome de fluor, un atome de chlore, un atome de brome, un atome d'iode, un groupe cyano, un groupe nitro, un groupe méthyle, un groupe éthyle, un groupe n-propyle, un groupe i-propyle, un groupe méthoxy, un groupe éthoxy, un groupe méthylthio, un groupe méthylsulfinyle, un groupe méthylsulfonyle, un groupe méthoxycarbonyle, un groupe éthoxycarbonyle, ou un radical $-(X)_n-R^8$,

X representant un atome d'oxygène, un atome de soufre, un groupe sulfinyle ou un groupe sulfonyle, n représentant un nombre de 0 ou 1, et

$R^8$ représentant un groupe trifluorométhyle, un groupe trichlorométhyle, un groupe dichlorofluorométhyle, un groupe difluorochlorométhyle, un groupe dichlorométhyle, un groupe chlorométhyle, un groupe difluorométhyle, un groupe pentafluoréthyle, un groupe tétrafluoréthyle, un groupe trifluorochloréthyle, un groupe trifluoréthyle, un groupe difluorodichloréthyle, un groupe trifluorodichloréthyle ou un groupe pentachloréthyle.

3. Procédé en vue de combattre les plantes adventices et les parasites de règne animal, caractérisé en ce qu'on fait agir des 4-nitro-1-phényl-pyrazoles de formule générale (I) selon la revendication 1, sur les plantes adventices et/ou les parasites du règne animal et/ou leur biotope, à l'exception des procédés non autorisés par la loi selon l'article 52 (4) CBE.

4. Utilisation de 4-nitro-1-phényl-pyrazoles de formule générale (I) selon la revendication 1, en vue de combattre les plantes adventices et/ou les parasistes du règne animal, à l'exception de l'utilisation en médecine humaine ou vétérinaire.

5. Procédé de préparation d'agents herbicides et insecticides, caractérisé en ce qu'on mélange des 4-nitro-1-phenyl-pyrazoles de formule générale (I) selon la revendication 1 avec des diluants et/ou des agents tensio-actifs.

6. 4-nitro-1-phényl-pyrazoles de formule générale (Ia):

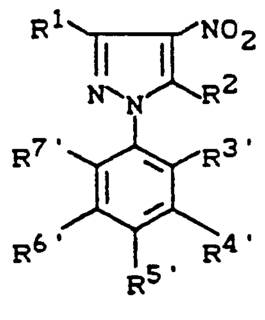

(Ia)

dans laquelle

$R^1$ et $R^2$ représentent chacun indépendamment l'un de l'autre un atome d'hydrogène ou un group halogénalkyle, un groupe alkylthioalkyle, un groupe alcoxyalkyle, un groupe hydroxyalkyle ou un groupe alkyle chaque fois à chaîne droite ou ramifiée et contenant chacun jusqu'à 4 atomes de carbone dans les fractions alkyle individuelles, et éventuellement jusqu'à 9 atomes d'halogènes indentiques ou différents, ou un groupe cycloalkyle contenant 3 à 7 atomes de carbone, et

$R^{3'}$, $R^{4'}$, $R^{5'}$, $R^{6'}$ et $R^{7'}$ représentent chacun indépendamment l'un de l'autre un atome d'hydrogène, un atome de fluor, un atome de chlore, un atome de brome, un atome d'iode, un groupe cyano, un groupe nitro, un groupe alcoxycarbonyle, un groupe alkylsulfonyle, un groupe alkylsulfinyle, un groupe alkylthio, un groupe alcoxy ou un groupe alkyle chaque fois à chaîne droite ou ramifiée contenant chacun jusqu'à 4 atomes de carbone dans les fractions alkyle individuelles, ou un radical $-(X)_n-R^8$,

X representant un atome d'oxygène, un atome de soufre, un groupe sulfinyle ou un groupe sulfonyle, n représentant un nombre de 0 ou 1, et

$R^8$ représentant un groupe halogénalkyle à chaîne droite ou ramifiée contenant jusqu'à 4 atomes de carbone et jusqu'à 9 atomes d'halogènes identiques ou différents,

cependant qu'au moins un des radicaux

$R^{3'}$, $R^{4'}$, $R^{5'}$, $R^{6'}$ ou $R^{7'}$ représente un groupe cyano, un groupe alkylthio, un groupe alkylsulfinyle, un groupe alkylsulfonyle ou un groupe alcoxycarbonyle contenant chacun 1 à 4 atomes de carbone dans les fractions alkyle individuelles, ou encore un radical $-(X)_n-R^8$ ou

au moins trois des radicaux

$R^{3'}$, $R^{4'}$, $R^{5'}$, $R^{6'}$ ou $R^{7'}$ représentant des atomes d'halogènes identiques ou différents cependant que, dans le cas où $R^1$ et $R^2$ représentent simultanément un atome d'hydrogène, au moins un des radicaux $R^{3'}$, $R^{4'}$, $R^{5'}$, $R^{6'}$ ou $R^{7'}$est différents du fluor.

7. Procédé de préparation de 4-nitro-1-phényl-pyrazoles de formule générale (Ia):

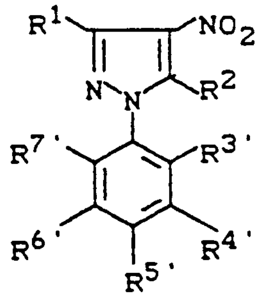

(Ia)

dans laquelle

$R^1$ et $R^2$ représentent chacun indépendamment l'un de l'autre un atome d'hydrogène ou un groupe halogénalkyle, un groupe alkylthioalkyle, un groupe alcoxyalkyle, un groupe hydroxyalkyle ou un groupe alkyle chaque fois à chaîne droite ou ramifiée contenant chacun jusqu'à 4 atomes de carbone dans les fractions alkyle individuelles, et éventuellement jusqu'à 9 atomes d'halogènes identiques ou différents, ou un groupe cycloalkyle contenant 3 à 7 atomes de carbone, et

$R^{3'}$, $R^{4'}$, $R^{5'}$, $R^{6'}$ et $R^{7'}$ représentant chacun indépendamment l'un de l'autre un atome d'hydrogène, un atome de fluor, un atome de chlore, un atome de brome, un atome d'iode, un groupe cyano, un groupe nitro, un groupe alcoxycarbonyle, un groupe alkylsulfonyle, un groupe alkylsulfinyle, un groupe alkylthio, un groupe alcoxy ou un groupe alkyle chaque fois à chaîne droite ou ramifiée contenant chacun jusqu'à 4 atomes de carbone dans les fractions alkyle individuelles, ou un radical $-(X)_n-R^8$,

X representant un atome d'oxygène, un atome de soufre, un groupe sulfinyle ou un groupe sulfonyle,

n représentant un nombre de 0 ou 1, et

$R^8$ représentant un groupe halogénalkyle à chaîne droite ou ramifiée contenant jusqu'à 4 atomes de carbone et jusqu'à 9 atomes d'halogènes identiques ou différents,

cependant qu'au moins un des radicaux

$R^{3'}$, $R^{4'}$, $R^{5'}$, $R^{6'}$ ou $R^{7'}$ représente un groupe cyano, un groupe alkylthio, un groupe alkylsulfinyle, un groupe alkylsulfonyle, ou un groupe alcoxycarbonyle contenant chacun 1 à 4 atomes de carbone dans les fractions alkyle individuelles, ou un radical $-(X)_n-R^8$ ou

au moins trois des radicaux

$R^{3'}$, $R^{4'}$, $R^{5'}$, $R^{6'}$ ou $R^{7'}$ représentent des atomes d'halogènes identiques ou différents, cependant que, dans le cas où $R^1$ et $R^2$ représentent simultanément un atome d'hydrogène, au moins un des radicaux $R^{3'}$, $R^{4'}$, $R^{5'}$, $R^{6'}$ ou $R^{7'}$ est différent de l'atome de fluor,

caractérisé en ce qu'on soumet, à une nitration, des 1-phenyl-pyrazoles de formule générale (II):

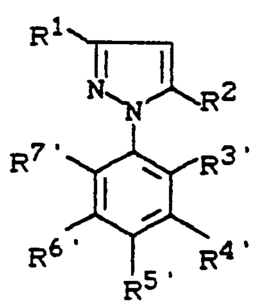

(II)

dans laquelle

$R^1$, $R^2$, $R^{3'}$, $R^{4'}$, $R^{5'}$, $R^{6'}$ et $R^{7'}$ ont les significations indiquées ci-dessus, avec de l'acide nitrique ou des sels d'acide nitrique, éventuellement en présence d'un diluant et éventuellement en présence d'un catalyseur.

## Claims

1. Herbicidal and insecticidal agents, characterized in that they contain at least one 4-nitro-1-phenyl-pyrazole of the general formula (I)

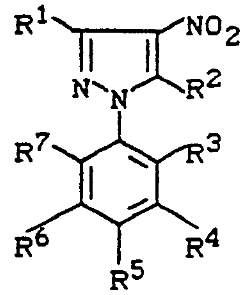

(I)

in which

$R^1$ and $R^2$ in each case independently of one another represent hydrogen, or represent in each case straight-chain or branched alkyl, hydroxyalkyl, alkoxyalkyl, alkylthioalkyl or halogenoalkyl with in each case up to 4 carbon atoms in the individual alkyl parts and, where appropriate, up to 9 identical or different halogen atoms, or represent cycloalkyl with 3 to 7 carbon atoms and

$R^3$, $R^4$, $R^5$, $R^6$ and $R^7$ in each case independently of one another represent hydrogen, fluorine, chlorine, bromine, iodine, cyano, nitro or in each case straight-chain or branched alkyl, alkoxy, alkylthio, alkylsulphinyl, alkylsulfonyl or alkoxycarbonyl with in each case up to 4 carbon atoms in the individual alkyl parts, or represent a radical $—(X)_n—R^8$,

wherein

X represents oxygen, sulphur, sulphinyl or sulphonyl,

n represents the number 0 or 1 and

$R^8$ represents straight-chain or branched halogeno-alkyl with up to 4 carbon atoms and up to 9 identical or different halogen atoms.

2. Herbicidal and insecticidal agents, characterized in that they contain at least one 4-nitro-1-phenyl-pyrazole of the general formula (I) according to Claim 1, in which

$R^1$ and $R^2$ independently of one another represent hydrogen, methyl, ethyl, n- or i-propyl, n-, i-, s- or t-butyl, cyclohexyl, hydroxymethyl, methoxymethyl, methylthiomethyl, trifluoromethyl or trichloromethyl and

$R^3$, $R^4$, $R^5$, $R^6$ and $R^7$ in each case independently of one another represent hydrogen, fluorine, chlorine, bromine, iodine, cyano, nitro, methyl, ethyl, n- or i-propyl, methoxy, ethoxy, methylthio, methylsulphinyl, methylsulphonyl, methoxycarbonyl or ethoxycarbonyl, or represent a radical $—(X)_n—R^8$,

wherein

X represents oxygen, sulphur, sulphinyl or sulphonyl,

n represents 0 or 1 and

$R^8$ represents trifluoromethyl, trichloromethyl, dichlorofluoromethyl, difluorochloromethyl, dichloromethyl, chloromethyl, difluoromethyl, pentachloromethyl, chloromethyl, difluoromethyl, pentafluoroethyl, tetrafluoroethyl, trifluorochloroethyl, trifluoroethyl, difluorodichloroethyl, trifluorodichloroethyl or pentachloroethyl.

3. Method of combating weeds and animal pests, characterized in that 4-nitro-1-phenyl-pyrazoles of the general formula (I) according to Claim 1 are allowed to act on weeds and/or animal pests and/or their environment, with the exception of the methods not permitted as stipulated by Article 52 (4) EPC.

4. Use of 4-nitro-1-phenyl-pyrazoles of the general formula (I) according to Claim 1 for combating weeds and/or animals pests, with the exception of the use in human or veterinary medicine.

5. Process for the preparation of herbicidal and insecticidal agents, characterized in that 4-nitro-1-phenyl-pyrazoles of the general formula (I) according to Claim 1 are mixed with extenders and/or surface-active agents.

6. 4-Nitro-1-phenyl-pyrazoles of the general formula (Ia)

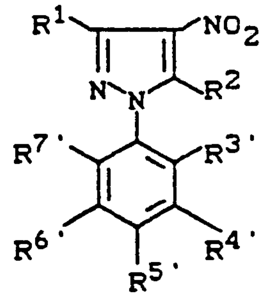

(Ia)

in which

$R^1$ and $R^2$ in each case independently of one another represent hydrogen, or represent in each case straight-chain or branched alkyl, hydroxyalkyl, alkoxyalkyl, alkylthioalkyl or halogenoalkyl with in each case up to 4 carbon atoms in the individual alkyl parts and, where appropriate, up to 9 identical or different halogen atoms, or represent cycloalkyl with 3 to 7 carbon atoms and

$R^{3'}$, $R^{4'}$, $R^{5'}$, $R^{6'}$ and $R^{7'}$ in each case independently of one another represent hydrogen, fluorine, chlorine, bromine, iodine, cyano, nitro or in each case straight-chain or branched alkyl, alkoxy, alkylthio, alkylsulphinyl, alkylsulfonyl or alkoxycarbonyl with in each case up to 4 carbon atoms in the individual alkyl parts, or represent a radical $-(X)_n-R^8$,

wherein

X represents oxygen, sulphur, sulphinyl or sulphonyl,

n represents the number 0 or 1 and

$R^8$ represents straight-chain or branched halogenoalkyl with up to 4 carbon atoms and up to 9 identical or different halogen atoms,

but wherein at least one of the radicals

$R^{3'}$, $R^{4'}$, $R^{5'}$, $R^{6'}$ or $R^{7'}$ represents cyano, or represents alkylthio, alkylsulphinyl, alkylsulphonyl or alkoxycarbonyl with in each case 1 to 4 carbon atoms in the individual alkyl parts, or represents a radical $-(X)_n-R^8$,

or wherein at least three of the radicals

$R^{3'}$, $R^{4'}$, $R^{5'}$, $R^{6'}$ or $R^{7'}$ represent identical or different halogen atoms, but in the case where $R^1$ and $R^2$ simultaneously represent hydrogen, at least one of the radicals $R^{3'}$, $R^{4'}$, $R^{5'}$, $R^{6'}$ or $R^{7'}$ is other than fluorine.

7. Process for the preparation of 4-nitro-1-phenyl-pyrazoles of the general formula (Ia)

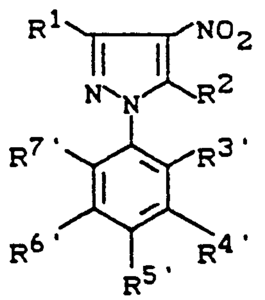

(Ia)

$R^1$ and $R^2$ in each case independently of one another represent hydrogen, or represent in each case straight-chain or branched alkyl, hydroxyalkyl, alkoxyalkyl, alkylthioalkyl or halogenoalkyl with in each case up to 4 carbon atoms in the individual alkyl parts and, where appropriate, up to 9 identical or different halogen atoms, or represent cycloalkyl with 3 to 7 carbon atoms and

$R^{3'}$, $R^{4'}$, $R^{5'}$, $R^{6'}$ and $R^{7'}$ in each case independently of one another represent hydrogen, fluorine, chlorine, bromine, iodine, cyano, nitro or in each case straight-chain or branched alkyl, alkoxy, alkylthio, alkylsulphinyl, alkylsulfonyl or alkoxycarbonyl with in each case up to 4 carbon atoms in the individual alkyl parts, or represent a radical $-(X)_n-R^8$,

wherein

X represents oxygen, sulphur, sulphinyl or sulphonyl,

n represents the number 0 or 1 and

$R^8$ represents straight-chain or branched halogenoalkyl with up to 4 carbon atoms and up to 9 identical or different halogen atoms,

but wherein at least one of the radicals

$R^{3'}$, $R^{4'}$, $R^{5'}$, $R^{6'}$ or $R^{7'}$ represents cyano, or represents alkylthio, alkylsulphinyl, alkylsulphonyl or alkoxycarbonyl with in each case 1 to 4 carbon atoms in the individual alkyl parts, or represents a radical $-(X)_n-R^8$,

or wherein at least three of the radicals

$R^{3'}$, $R^{4'}$, $R^{5'}$, $R^{6'}$ or $R^{7'}$ represent identical or different halogen atoms, but in the case where $R^1$ and $R^2$ simultaneously represent hydrogen, at least one of the radicals $R^{3'}$, $R^{4'}$, $R^{5'}$, $R^{6'}$ or $R^{7'}$ is other than fluorine, characterized in that 1-phenylpyrazoles of the general formula (II)

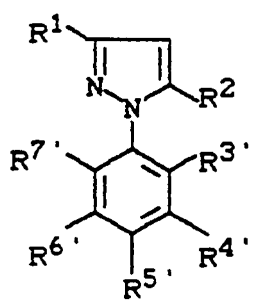

(II)

in which

$R^1$, $R^2$, $R^{3'}$, $R^{4'}$, $R^{5'}$, $R^{6'}$ and $R^{7'}$ have the following abovementioned meaning, are nitrated with nitric acid or nitric acid salts, if appropriate in the presence of a diluent and if appropriate in the presence of a catalyst.